(19)

**Europäisches Patentamt**

**European Patent Office**

**Office européen des brevets**

(11) **EP 3 699 223 A1**

(12) **EUROPÄISCHE PATENTANMELDUNG**

(43) Veröffentlichungstag:
**26.08.2020 Patentblatt 2020/35**

(21) Anmeldenummer: **19157923.4**

(22) Anmeldetag: **19.02.2019**

(51) Int Cl.:
*C08K 5/00* (2006.01)  *C08K 9/06* (2006.01)
*G01N 29/04* (2006.01)  *C08C 19/25* (2006.01)
*C08K 5/5419* (2006.01)  *C08K 5/54* (2006.01)
*C08K 3/013* (2018.01)  *B29C 48/92* (2019.01)
*C08K 3/22* (2006.01)  *C08K 3/26* (2006.01)
*C08K 3/34* (2006.01)  *C08K 3/36* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **LANXESS Deutschland GmbH**
**50569 Köln (DE)**

(72) Erfinder:
• **SCHRÖDER, Andreas**
**69469 Weinheim (DE)**
• **GRÄFF, Ludwig**
**68519 Viernheim (DE)**
• **SCHILLING, Jürgen**
**68723 Schwetzingen (DE)**
• **WAWRZINSKI, Lars**
**68309 Mannheim (DE)**

(54) **INLINE ULTRASCHALLKONTROLLE ZUR DETEKTION DES ANVERNETZENS EINER KAUTSCHUKMISCHUNG BEI DER IN SITU SILANISIERUNG VON HELLEN FÜLLSTOFFEN**

(57)    Die Erfindung betrifft ein Verfahren zur Prüfung der Silanisierung von hellen Füllstoffen, wobei eine Mischung enthaltend mindestens einen silanisierten hellen Füllstoff, vorzugsweise silanisierte Kieselsäure, und mindestens einen Kautschuk mit Ultraschallwellen in einem Frequenzbereich von 4 bis 10 MHz, vorzugsweise von 5 bis 7 MHz, beschallt wird und die Signalstärke der Ultraschallwellen nach Transmission durch die Kautschukmischung bestimmt wird, wobei der relative Dämpfungskoeffizient $\alpha_{rel}$ der Kautschukmischung in dem Frequenzbereich der Ultraschallwellen bestimmt wird, die Standardabweichung $\sigma$ des relativen Dämpfungskoeffizienten $\alpha_{rel}$ ermittelt wird und $\alpha_{rel}$ und $\sigma$ zur Detektion des Anvernetzens der Kautschukmischung verwendet werden.

EP 3 699 223 A1

**Beschreibung**

Die Erfindung betrifft das technische Gebiet der Silanisierung von hellen Füllstoffen. Unter hellen Füllstoffen versteht der Fachmann mineralische natürliche und synthetische Füllstoffe, welche nicht auf Ruß basieren, wie z.B. Glimmer, Kaoline, Kreiden, Calciumcarbonate, Talk, Zinkoxide, Aluminiumoxide, Titandioxide, Kieselsäure und Silikate. Zur Verbesserung der Verarbeitbarkeit werden die hellen Füllstoffe durch Silanisierung hydrophobiert, wodurch sich die Eigenschaften der elastomeren Vulkanisate deutlich verbessern. Insbesondere in Kautschukmischungen für Reifenlaufflächen führt die Verwendung von silanisierten Füllstoffen im Vergleich zum schwarzen Füllstoff Ruß zu einer Verbesserung der Nassrutschfestigkeit und des Rollwiderstands, ohne den Abrieb des Reifens zu erhöhen.

Bei der Herstellung von z.B. Reifenlaufflächenmischungen wird die Kieselsäure mit einem Silanisierungsmittel in einem ersten Mischschritt im Kautschuk in einem Innenmischer diskontinuierlich dispergiert. Der Mischprozess wird dabei durch Einstellung der Mischparameter (Drehzahl, Füllgrad, Temperierung, etc.) so gefahren, dass die Auswurftemperatur im Bereich von 140°C bis 160°C liegt (vgl. F. Röthemeyer, F. Sommer; "Kautschuktechnologie Werkstoffe, Verarbeitung Produkte", Hanser Verlag 2. überarbeitete Auflage; ISBN 10: 3-446-40480-5; Seite 272). In einem zweiten Mischschritt im Innenmischer wird die Mischung im Bereich von 140°C bis 160°C homogenisiert. Unterhalb von 140°C findet keine Reaktion des Silans mit der Kieselsäure statt, oberhalb von 160°C kann das Silan mit dem Kautschuk anvernetzten. Als Innenmischer kommen in der Gummiindustrie auch Innenmischer mit einem Austragsextruder zur Anwendung. Neben diskontinuierlichen Herstellungsverfahren werden auch kontinuierliche Verfahren mit Extrudern getestet, wie in der DE69728538T2 offenbart. Die Silanisierung in einer Kautschukmischung wird als in situ Silanisierung bezeichnet. Dieses ist beispielsweise beschrieben in EP-A-0911359 wie auch in EP-A-1 357156.

Da die Temperaturen und Mischzeiten bei der in situ Silanisierung exakt eingehalten werden müssen (vgl. F. Röthemeyer, F. Sommer; "Kautschuktechnologie Werkstoffe, Verarbeitung Produkte", Hanser Verlag 2. überarbeitete Auflage; ISBN 10: 3-446-40480-5; Seite 272), sind diese Verfahren sehr störanfällig. Prüfungen sollten daher möglichst schnell und während des Herstellprozesses (inline), volumetrisch und repräsentativ durchgeführt werden. Nur in diesem Fall kann die in situ Silanisierung zuverlässig gesteuert, die thermische Belastung der Kautschukmischung niedrig und die Verfahrenseffizienz hoch gehalten und die Ausschussraten reduziert werden. Dies gilt umso mehr beim kontinuierlichen Verfahren zur in situ Silanisierung, welche sich auch wegen der unzureichenden Möglichkeit der inline Prüfung noch nicht auf dem Markt durchsetzen konnte.

Entgegen den Anforderungen zur Kontrolle der in situ Silanisierung können Messungen von unvernetzten Kautschukmischungen in der Gummiindustrie in der Regel erst mehrere Minuten oder sogar Tage nach der Herstellung der Kautschukmischung durchgeführt werden (sogenannte offline Verfahren), da die Messungen zeitintensiv sind und eine zeitgleiche Messung von mehreren Proben eine Vielzahl teurer Messapparaturen erfordert. Daher wird die Qualität einer unvernetzten Kautschukmischung bislang lediglich stichprobenartig (< 0,1% der Gesamtmenge) überprüft. Typischerweise wird die MooneyViskosität der Stichproben der Kautschukmischung ermittelt, um die Vollständigkeit der Silanisierungsreaktion zu überprüfen (vgl. F. Röthemeyer, F. Sommer; "Kautschuktechnologie Werkstoffe, Verarbeitung Produkte", Hanser Verlag 2. überarbeitete Auflage; ISBN 10: 3-446-40480-5; Seite 390). Die geringe Anzahl der Stichproben ist jedoch nicht repräsentativ für die gesamte Kautschukmischung.

Für die Analyse von mit Extrudern compoundierten Kunststoffen sind eine Vielzahl repräsentativer inline Qualitätskontrollen bekannt, zum Beispiel NIR, Raman und UV/VIS Spektroskopie, bei denen die Proben durchstrahlt werden (vgl. Hochrein et al., Plastverarbeiter, Sept. 2009, S. 92). Bei Verwendung elektromagnetischer Wellen stört jedoch der Ruß, da die elektromagnetischen Wellen schon bei sehr geringen Anteilen von Ruß nahezu vollständig absorbiert werden. Bei Verwendung von mechanischen Druckwellen im hohen Frequenzbereich > 1 MHz (Ultraschallwellen) stört Ruß hingegen vergleichsweise wenig. Hier sind jedoch helle Füllstoffe mit einer hohen Dichte, wie Kieselsäure, Kaolin oder Kreide, störend (vgl. A. Schröder, L. Gräff, L. Wawrzinski, Rubber World 251 (2015) 28), da diese die Ultraschallwellen stärker dämpfen als Ruß mit einer niedrigeren Dichte. Zudem besitzen unvernetzte Kautschuke im Gegensatz zu Kunststoffen aufgrund ihrer viskoelastischen Eigenschaften an sich schon eine relative hohe Dämpfung. Aufgrund dieser hohen Dämpfung gibt es bei volumetrischen Messungen in Transmission eine obere Grenze für den Abstand zwischen dem sendenden und dem empfangenden Ultraschallwandler. Wird diese Grenze überschritten, ist das Signal/Rauschverhältnis zu schlecht, um verwertbare Messwerte zu erhalten.

Auf der anderen Seite gibt es eine untere Grenze für den Abstand zwischen den Ultraschallwandlern, da die hochviskose Kautschukmischung noch zwischen den Ultraschallwandlern hindurch fließen muss. So wird in Rubber World 251 (2015) 28 die Verwendung von Ultraschall als inline Qualitätskontrolle für die Detektion von variierenden Anteilen an Füllstoff und Vernetzerbatches mit Markern sowie der Detektion von Verunreinigungen größer als 100 $\mu$m und der Makrodispersion von Füllstoffen beschrieben, allerdings nur bis zu einem Anteil von 30 phr an hellen Füllstoffen. Der Anteil an hellen Füllstoffen in Kautschukmischungen für Reifenlaufflächen liegt in der Regel bei mindestens 50 phr, was zu einer Zunahme der Viskosität der Kautschukmischung führt.

Die Verfolgung einer chemischen Reaktion, nämlich die Vernetzung eines unvernetzten Kautschuks mit Schwefel, wird in M. Jaunich, B. Stark, B. Hoster, Polymer Testing 28 (2009) 84 beschrieben. Hierbei wird die Änderung der Vernetzungsdichte mit Hilfe der Schallgeschwindigkeit verfolgt. Dabei werden Ultraschallwandler mit einer Mittenfrequenz von f=4 MHz verwendet. Eine Extrusion der Mischung findet nicht statt.

In WO2017129471 A1 wird ein Verfahren zur Prüfung der Silanisierung offenbart, welches eine inline Kontrolle der in situ Silanisierung von hellen Füllstoffen, insbesondere von gefällten Kieselsäuren, erlaubt. Das darin offenbarte Verfahren ist ein Verfahren zur Kontrolle der Effektivität von Silanisierungsreaktionen. Die Silanisierung wird dabei bei Temperaturen unterhalb von 160°C durchgeführt. Höhere Temperaturen ermöglichen jedoch höhere Durchsätze der Kautschukmischungen bei gleichbleibender Zeitspanne, und ein höherer Durchsatz ermöglicht eine schnellere und damit kostengünstigere Herstellung der Kautschukmischungen. Jedoch besteht bei Durchführung der Silanisierung bei höheren Temperaturen die Gefahr, dass die Kautschukmischung unerwünscht anvernetzt (auch als "Anscorchen" bezeichnet) und/oder sich das Silanisierungsmittel zersetzt. In der Praxis ist die Gefahr einer solchen Anvernetzung wesentlich größer als eine ineffektive Silanisierung einer Füllstoffoberfläche bei geringeren Temperaturen. Ein Anvernetzen der Kautschukmischung ist unerwünscht, da dabei die Fließfähigkeit der Kautschukmischung eingeschränkt wird, was zu Problemen bei der Weiterverarbeitung der Kautschukmischung führt.

Es bestand daher Bedarf an einem inline Verfahren zur Prüfung der in situ Silanisierung von hellen Füllstoffen, insbesondere von Kieselsäuren, welches eine möglichst kostengünstige Herstellung von silanisierten hellen Füllstoffen enthaltenden Kautschukmischungen ermöglicht.

Kostengünstige Verfahren zur Herstellung von Kautschukmischungen mit silanisierten hellen Füllstoffen sind vorzugsweise Verfahren mit einem hohen Durchsatz, vorzugsweise gekennzeichnet durch Herstellung bei einer Temperatur von größer als 160°C.

Daher sollte das Verfahren zur Prüfung der in situ Silanisierung von hellen Füllstoffen ermöglichen, silanisierte helle Füllstoffe enthaltende Kautschukmischungen vorzugsweise mit einem hohen Durchsatz herstellen zu können, besonders bevorzugt bei einer Temperatur von größer als 160°C.

Weiter bevorzugt sollte das Verfahren zur Prüfung der in situ Silanisierung von hellen Füllstoffen ermöglichen, das Anvernetzen der Kautschukmischung zu detektieren.

Vorzugsweise sollte das Ergebnis der Prüfung der Silanisierung spätestens eine Minute nach Austritt aus dem Extruder vorliegen, um so eine umgehende Rückkopplung zur Verfahrenssteuerung zu erlauben und eine Steigerung der Produktivität und/oder eine Verringerung der Ausschussraten des Prozesses zu ermöglichen. Bevorzugt sollte das Verfahren auch eine repräsentative Kontrolle erlauben, d.h. eine Prüfung von wenigstens 1% der mindestens einen silanisierten hellen Füllstoff enthaltenden Kautschukmischung ermöglichen, vorzugsweise unter Produktionsbedingungen, d.h. bei Durchsätzen von $\geq$ 5 kg/h. Von besonderem Vorteil wäre dabei ein zerstörungsfrei erfolgendes Verfahren. Um eine Verwendung des Verfahrens bei der Produktion von Kautschukmischungen für Reifen zu ermöglichen, wäre es wünschenswert, dass Ruß als zusätzlicher Bestandteil der Kautschukmischung die Qualitätskontrolle nur in geringem Umfang, bevorzugt nicht stört.

## Verfahren zur Prüfung der Silanisierung von hellen Füllstoffen

Überraschend wird die Aufgabe gelöst durch ein Verfahren zur Prüfung der Silanisierung von hellen Füllstoffen, wobei eine Mischung enthaltend mindestens einen silanisierten hellen Füllstoff, vorzugsweise silanisierte Kieselsäure, und mindestens einen Kautschuk, mit Ultraschallwellen in einem Frequenzbereich von 4 bis 10 MHz, vorzugsweise von 5 bis 7 MHz, beschallt wird und die Signalstärke der Ultraschallwellen nach Transmission durch die Kautschukmischung bestimmt wird,
wobei der relative Dämpfungskoeffizient $\alpha_{rel}$ der Kautschukmischung in dem Frequenzbereich der Ultraschallwellen bestimmt wird, die Standardabweichung $\sigma$ des relativen Dämpfungskoeffizienten $\alpha_{rel}$ ermittelt wird und $\alpha_{rel}$ und $\sigma$ zur Detektion des Anvernetzens der Kautschukmischung verwendet werden.

Bevorzugt wird in dem Verfahren zur Prüfung der Silanisierung von hellen Füllstoffen die Mischung enthaltend mindestens einen silanisierten hellen Füllstoff, vorzugsweise silanisierte Kieselsäure, und mindestens einen Kautschuk, extrudiert und das erhaltene Extrudat wie vorstehend beschrieben beschallt. Weiter bevorzugt erfolgt die Beschallung des Extrudats in einem Extruder.

Unter Extrudat wird im Sinne der vorliegenden Erfindung sowohl die extrudierende Mischung enthaltend mindestens einen silanisierten hellen Füllstoff und mindestens einen Kautschuk verstanden als auch die bereits extrudierte Mischung enthaltend mindestens einen silanisierten hellen Füllstoff und mindestens einen Kautschuk. Bevorzugt ist das Extrudat im Sinne der vorliegenden Erfindung die extrudierende Mischung enthaltend mindestens einen silanisierten hellen Füllstoff und mindestens einen Kautschuk.

Bevorzugt wird die Kautschukmischung bei einer Temperatur von größer als 160°C hergestellt, besonders bevorzugt bei einer Temperatur von größer als 160°C bis 200°C, ganz besonders bevorzugt bei einer Temperatur von größer als 160°C bis 180°C.

Bevorzugt liegt die Temperatur der Kautschukmischung während der Beschallung oberhalb des Glasübergangsbereiches der Kautschukmischung im Frequenzbereich der Ultraschallwellen. Bevorzugt liegt die Temperatur der Kautschukmischung während der Beschallung bei mindestens 80°C, besonders bevorzugt bei mindestens 100°C.

Unter Anvernetzung der Kautschukmischung wird im Sinne der vorliegenden Erfindung verstanden, dass sich kovalente Bindungen, bevorzugt schwefelhaltige kovalente Bindungen, zwischen den Polymerketten des mindestens einen Kautschuks und/oder zwischen den Polymerketten des mindestens einen Kautschuks und dem mindestens einen silanisierten hellen Füllstoff ausgebildet haben. Sofern die Kautschukmischung mindestens ein Silanisierungsmittel enthält, wird unter Anvernetzung im Sinne der vorliegenden Erfindung auch die Ausbildung kovalenter Bindungen, bevorzugt schwefelhaltiger kovalenter Bindungen, zwischen den Polymerketten des mindestens einen Kautschuks und dem mindestens einen Silanisierungsmittel verstanden.

Die Anvernetzung der Kautschukmischung kann durch verschiedene Parameter detektiert werden. Bevorzugt wird die Anvernetzung der Kautschukmischung durch den relativen Dämpfungskoeffizienten $\alpha_{rel}$ der Kautschukmischung in dem Frequenzbereich der Ultraschallwellen und seiner Standardabweichung $\sigma$ detektiert.

Besonders bevorzugt wird die Anvernetzung der Kautschukmischung durch den relativen Dämpfungskoeffizienten $\alpha_{rel}$ der Kautschukmischung in dem Frequenzbereich der Ultraschallwellen und seiner gleitenden Standardabweichung $\sigma_g$ detektiert.

Bevorzugt wird das Anvernetzen einer Kautschukmischung detektiert, die bei einer Temperatur von größer als 160°C hergestellt wird.

Bevorzugt wird die Standardabweichung $\sigma$ des relativen Dämpfungskoeffizienten $\alpha_{rel}$ der anvernetzten Kautschukmischung auf eine Messzeit von 1 bis 500 s bezogen, besonders bevorzugt von 1 bis 100 s, ganz besonders bevorzugt 1 bis 50 s, meist bevorzugt von 1 bis 15 s.

Besonders bevorzugt wird die gleitende Standardabweichung $\sigma_g$ des relativen Dämpfungskoeffizienten $\alpha_{rel}$ der anvernetzten Kautschukmischung auf eine Messzeit von 1 bis 100 s bezogen, besonders bevorzugt von 1 bis 50 s, ganz besonders bevorzugt von 1 bis 15 s.

Bevorzugt wird das Anvernetzen der Kautschukmischung dadurch detektiert, dass die Standardabweichung $\sigma$ des relativen Dämpfungskoeffizienten $\alpha_{rel}$ der anvernetzten Kautschukmischung, bevorzugt hergestellt bei einer Temperatur von größer als 160°C, größer als 0,5 [1/m], bevorzugt größer als 1 [1/m] ist, bevorzugt bezogen auf eine Messzeit von 1 bis 500 s, besonders bevorzugt 1 bis 100 s, ganz besonders bevorzugt von 1 bis 50 s, ganz besonders bevorzugt 1 bis 15 s, und der relative Dämpfungskoeffizient $\alpha_{rel}$ der anvernetzten Kautschukmischung, bevorzugt hergestellt bei einer Temperatur von größer als 160°C, kleiner als 0 [1/m] ist, besonders bevorzugt kleiner als -15 [1/m].

Besonders bevorzugt wird das Anvernetzen der Kautschukmischung dadurch detektiert, dass die gleitende Standardabweichung $\sigma_g$ des relativen Dämpfungskoeffizienten $\alpha_{rel}$ der anvernetzten Kautschukmischung, hergestellt bei einer Temperatur von größer als 160°C, größer als 1 [1/m] ist, bezogen auf eine Messzeit von 1 bis 50 s, bevorzugt von 1 bis 15 s, und der relative Dämpfungskoeffizient $\alpha_{rel}$ der anvernetzten Kautschukmischung, hergestellt bei einer Temperatur von größer als 160°C, kleiner als 0 [1/m] ist, bevorzugt kleiner als -15 [1/m].

Bevorzugt wird die Anvernetzung der Kautschukmischung im Sinne der vorliegenden Erfindung dadurch gekennzeichnet, dass die Kautschukmischung mit fortschreitender Anvernetzung eine höhere Viskosität aufweist.

Bevorzugt ist die Viskosität einer anvernetzten Kautschukmischung nach Mooney ML1+4 (100°C) um mindestens 5 MU (Mooney Units) größer als die einer noch nicht anvernetzten Referenzkautschukmischung mit gleicher Zusammensetzung.

Unter einer noch nicht anvernetzten Referenzkautschukmischung wird im Sinne der vorliegenden Erfindung eine Kautschukmischung verstanden, die nicht anvernetzt vorliegt, die die gleiche Zusammensetzung aufweist und bei einer Temperatur von mindestens 140°C bis 160°C hergestellt wurde, bevorzugt im gleichen Mischaggregat, besonders bevorzugt mit gleicher Anzahl an Mischstufen, verglichen mit einer anvernetzten Kautschukmischung, für die sie als Referenz dient. Besonders bevorzugt liegen in der noch nicht anvernetzten Referenzkautschukmischung mehr als 90 Gew.-% des mindestens einen hellen Füllstoffs, bevorzugt mehr als 95 Gew.-%, besonders bevorzugt mehr als 99 Gew.-%, silanisiert vor.

Weiter bevorzugt wird die Ultraschallamplitude der noch nicht anvernetzten Referenzkautschukmischung bei gleicher Temperatur, gleichem Druck, und gleicher Scherrate ermittelt wie die der anvernetzten Kautschukmischung.

Bevorzugt ist im Sinne dieser Erfindung der Speicher Modul G', gemessen mit dem RPA 2000 der Firma Alpha Technologies bei 1% Dehnungsamplitude bei einer Frequenz von 1 Hz und einer Temperatur von 60°C, der anvernetzten Kautschukmischung gleich groß oder kleiner als der Modul G' der noch nicht anvernetzten Referenzkautschukmischung, besonders bevorzugt kleiner.

Bevorzugt ist das erfindungsgemäße Verfahren ein Verfahren zur Detektion des Anvernetzens der Kautschukmischung.

Bei dem erfindungsgemäßen Verfahren handelt es sich bevorzugt um ein inline-Verfahren, da die Prüfung der Silanisierung während des Herstellprozesses der Kautschukmischung erfolgt.

Bevorzugt endet der Herstellprozess der Kautschukmischung nach dem Austritt des Extrudats aus dem Extruder.

Als Kautschuke eignen sich insbesondere Naturkautschuk (NR), Isoprenkautschuk (IR), Styrol-Butadien-Kautschuk (SBR), Butadienkautschuk (BR), Isopren-Isobutylen-Kautschuk (IIR), Polychloroprenkautschuk (CR), Acrylnitril-Butadien-Kautschuk (NBR), hydrierter Acrylnitril-Butadien-Kautschuk (HNBR), carboxylierter Acrylnitril-Butadien-Kautschuk (XNBR), hydrierter carboxylierter Acrylnitril-Butadien-Kautschuk (HXNBR), Ethylen-Propylen-Dien-Kautschuk (EPDM), Ethylen-Propylen-Kautschuk (EPM), Fluorkautschuk (FKM), perfluorierter Fluorkautschuk (FFKM), Acrylat-Ethylen-Kautschuk (AEM), Acrylatkautschuk (ACM), Ethylen-Methylen-Acrylatkautschuk (EMA), chloriertes Polyethylen, chlorsulfoniertes Polyethylen, Ethylen-Vinylacetat-Kautschuk (EVA), Siliconkautschuk, Fluro-Silicon-Kautschuk, Ethylen-Epichlorhydrin Kautschuk (ECO), Epichlorhydrin-Kautschuk (CO) und/oder Polyurethan-Kautschuk (PU).

Besonders bevorzugt ist der mindestens eine Kautschuk ausgewählt aus der Gruppe bestehend aus Naturkautschuk (NR), Isoprenkautschuk (IR), Styrol-Butadien-Kautschuk (SBR) und Butadienkautschuk (BR), ganz besonders bevorzugt Styrol-Butadien-Kautschuk (SBR) und Butadienkautschuk (BR).

Bevorzugte helle Füllstoffe im Sinne der Erfindung sind Glimmer, Kaolin, Kieselerde, Calciumcarbonate, Zinkoxide, Aluminiumoxide, Titandioxide, Kieselsäuren, Kreide und Talkum, besonders bevorzugt Kieselsäuren und meist bevorzugt gefällte Kieselsäuren.

In einer speziellen Ausführungsform der vorliegenden Erfindung können sogenannte Dual Phase Fillers als heller Füllstoff eingesetzt werden. Dabei handelt es sich um Rußpartikel mit einem Anteil von > 5% an Kieselsäuren, welche dem Fachmann bekannt sind.

Bei der Silanisierung handelt es sich um eine chemische Anbindung eines Silans an die Oberfläche des hellen Füllstoffs. Die Anbindung erfolgt durch Kondensationsreaktionen zwischen hydrolysierbaren Gruppen der verwendeten Silane und chemischen Gruppen an der Oberfläche des hellen Füllstoffs.

Bevorzugt wird der mindestens eine silanisierte helle Füllstoff aus mindestens einem hellen Füllstoff und mindestens einem Silanisierungsmittel hergestellt.

In einer bevorzugten Ausführungsform erfolgt die Herstellung des mindestens einen silanisierten hellen Füllstoffs aus mindestens einem hellen Füllstoff und mindestens einem Silanisierungsmittel in einem Extruder.

Bevorzugt wird als das mindestens eine Silanierungsmittel ein Silan verwendet. Bevorzugt als Silan sind bifunktionelle Silane, bei denen eine funktionelle Gruppe mit der Oberfläche eines hellen Füllstoffs reagieren kann, z.B. Trialkoxsilygruppen, und die andere funktionelle Gruppe an der Vernetzungsreaktion des Kautschuks teilnehmen kann, wie zum Beispiel Thiol oder Polysulfangruppen.

Besonders bevorzugt sind folgende Silane:

Bis(3-triethoxysilylpropyl)tetrasulfid (beispielsweise am Markt erhältlich als Si69® der Firma Evonik Industries AG), Bis(triethoxysilylpropyl)disulfid (beispielsweise am Markt erhältlich als Si75® der Firma Evonik Industries AG), Bis(triethoxysilylpropyl)disulfid (beispielsweise am Markt erhältlich als Si266® der Firma Evonik Industries AG), 3-Thiocyanatopropyltriethoxysilan (beispielsweise am Markt erhältlich als Si264™ der Firma Evonik Industries AG), 3-Mercaptopropyl-di(tridecan-1-oxy-13-penta(ethylenoxid))ethoxysilan (beispielsweise am Markt erhältlich als VP Si 363® der Firma Evonik Industries AG) und Gammamercaptopropyltrimethoxysilan.

Ganz besonders bevorzugt ist das Silan Bis(3-triethoxysilylpropyl)tetrasulfid.

Die Kautschukmischung kann neben dem mindestens einen silanisierten hellen Füllstoff und dem mindestens einen Kautschuk mindestens einen hellen Füllstoff und/oder mindestens ein Silanisierungsmittel enthalten.

Bei Fortschreiten der Silanisierung des mindestens einen hellen Füllstoffs nimmt der Anteil an mindestens einem hellen Füllstoff und mindestens einem Silanisierungsmittel in der Kautschukmischung ab und der Anteil an mindestens einem silanisierten hellen Füllstoff nimmt zu.

Da der mindestens eine silanisierte helle Füllstoff und das mindestens eine Silanisierungsmittel fähig sind, den Kautschuk anzuvernetzen, können sie als Vernetzungsmittel verstanden werden.

Bevorzugt enthält die Kautschukmischung, welche bevorzugt weniger als 10 phr Vernetzungsmittel außer dem mindestens einen Silanisierungsmittel und dem mindestens einen silanisierten hellen Füllstoff enthält, besonders bevorzugt weniger als 5 phr, ganz besonders bevorzugt weniger als 2 phr. Meist bevorzugt weist die Kautschukmischung keine anderen Vernetzungsmittel außer dem mindestens einen Silanisierungsmittel und dem mindestens einen silanisierten hellen Füllstoff auf.

Der Anteil an hellen Füllstoffen in der Kautschukmischung beträgt typischerweise 20 bis 250 phr, vorzugsweise 30 bis 150 phr und besonders bevorzugt 40 bis 100 phr.

In einer Ausführungsform des erfindungsgemäßen Verfahrens werden die Kautschukmischungen mit mindestens einem Paar, vorzugsweise mit mindestens einem Detektionsband von wenigstens zwei Paaren Ultraschallwandlern analysiert. Die jeweiligen Wandler eines Paares sind gegenüberliegend und die Paare in Reihe nebeneinander angeordnet.

Ein Ultraschallwandler eines Paares fungiert dabei als Sender und der andere Wandler als Empfänger des Ultraschallsignals. Im Sender werden Spannungspulse, generiert in der Prüfelektronik, mit Hilfe des piezoelektrischen Effektes in Ultraschallwellen umgewandelt. Typerweise weist ein Spannungspuls bei der Anregung des Senders die Form eines Rechteckpulse oder eines Nadelpulses auf. Bevorzugt werden mindestens zwei Spannungspulse (Burst-Signal) hintereinander mit einem zeitlichen Abstand von $\geq 0,1 \mu s$ und $\leq 1 \mu s$ verwendet, besonders bevorzugt werden mindestens drei Spannungspulse, ganz besonders bevorzugt mindestens sieben Spannungspulse verwendet. Besonders bevorzugt entspricht der reziproke zeitliche Abstand zwischen den Spannungspulsen eines Burst-Signales der Eigenfrequenz der verwendeten Ultraschallwandler. Das Aussenden eines Ultraschallsignales bestehend aus einem einzelnen Spannungspuls bzw. aus Burst-Signalen wird bevorzugt mit einer Frequenz von mindestens 1 Hz wiederholt, besonders bevorzugt mindestens 10 Hz. Im Empfänger werden die Ultraschallwellen während der Extrusion nach Durchschallung (Transmission) durch die Kautschukmischung wieder in Spannungssignale umgewandelt und können zeitnah, bevorzugt in weniger als 1 min, ausgewertet werden.

Im Spalt zwischen den Ultraschallwandlern befindet sich die Kautschukmischung. Dabei besteht vorzugsweise ein direkter Kontakt zwischen den Ultraschallwandlern und der Kautschukmischung. Der Spalt ist vorzugsweise an den Seiten begrenzt, wodurch ein Fließkanal mit einer Höhe von $\geq 5$ mm, besonders bevorzugt mit einer Höhe von $\geq 10$ mm entsteht. Vorzugsweise beträgt die Breite des Fließkanals $\geq 15$ mm, besonders bevorzugt $\geq 20$ mm. Die Kautschukmischung durchläuft mit einer Geschwindigkeit von vorzugsweise 0,1 m/min oder mehr, besonders bevorzugt 1 m/min oder mehr, ganz besonders bevorzugt 10 m/min oder mehr das Detektionsband. Hierfür wird in einer bevorzugten Ausführungsform ein Extruder verwendet. Bevorzugt ist der Fließkanal ein Bestandteil des Extruders. Dabei wird ein Teil der Kautschukmischung, vorzugsweise mehr als 1 Vol.-%, bevorzugt mehr als 10 Vol.-% besonders bevorzugt mehr als 25 Vol.-% der Kautschukmischung mit dem Ultraschall analysiert. Auf diese Weise ist eine repräsentative und nicht nur stichprobenartige Prüfung der Silanisierung von Kautschukmischungen möglich.

Bevorzugt beträgt der Abstand zwischen einem Sender, der die Ultraschallwellen emittiert und einem Empfänger, der die Ultraschallwellen empfängt, mindestens 5 mm, besonders bevorzugt 5 mm bis 15 mm.

Bevorzugt beträgt der Druck in der Kautschukmischung zwischen den Schallwandlern während der Beschallung mindestens 10 bar, besonders bevorzugt mindestens 30 bar, ganz besonders bevorzugt zwischen 50 und 100 bar.

Bevorzugt werden Ultraschallwellen im Frequenzbereich von 5 bis 7 MHz verwendet. Zur Messung können Ultraschalwellen einer oder mehrerer Frequenzen f verwendet werden. Bei der Beaufschlagung mit einem Frequenzspektrum ist die Verwendung von Ultraschallwellen im Bereich von z.B. von 5 bis 7 MHz typischerweise so zu verstehen, dass bei der emittierten Ultraschallfrequenz f das arithmetische Mittel der Grenzfrequenzen, also der unteren und der oberen Frequenz, bei der die Ultraschallwellenamplitude bzw. die Ultraschallwellenintensität 50% des Maximalwerts beträgt, von 5 bis 7 MHz liegt.

In einer bevorzugten Ausführungsform der Erfindung wird die Amplitude der in Spannungssignale umgewandelten Schallwellen in Abhängigkeit der Zeitspanne seit dem Aussenden des Schallsignales im Sender gemessen. Diese sogenannte A-Scans werden in Abhängigkeit der Zeit registriert.

In einer Ausführungsform der Erfindung wird zur Quantifizierung der Signalstärke der Ultraschallwellen aus dem Ultraschallsignal eines A-Scans der Wert einer Ultraschallwellenamplitude, vorzugsweise die maximale Ultraschallwellenamplitude, ermittelt und der Logarithmus der Ultraschallwellenamplitude a gegen die Zeit t dargestellt.

Vorzugsweise wird das Ultraschallsignal eines A-Scans und nicht die Echos des Ultraschallsignales ausgewertet.

In einer weiteren Ausführungsform der Erfindung wird durch Quadrieren der Ultraschallwellenamplitude a die Ultraschallwellenintensität i ermittelt und der Logarithmus der Ultraschallwellenintensität i gegen die Zeit t dargestellt.

Bevorzugt wird der relative Dämpfungskoeffizient $\alpha_{rel}$ gemäß der Formel (1a)

$$\alpha_{rel} = (\ln a_{ref} - \ln a)/x = (\ln i_{ref} - \ln i)/2x \qquad (1a)$$

aus der Ultraschallwellenamplitude a und der Ultraschallwellenamplitude $a_{ref.}$ einer Referenz und dem Abstand x zwischen den Ultraschallwandlern ermittelt. Alternativ kann der relative Dämpfungskoeffizient $\alpha_{rel}$ aus der Ultraschallwellenintensität i, Ultraschallwellenintensität $i_{ref}$ einer Referenz und dem Abstand x zwischen den Ultraschallwandlern ermittelt werden.

Als Referenz wird bevorzugt eine Kautschukmischung mit gleicher Zusammensetzung eingesetzt wie die zu überprüfende Kautschukmischung.

Ganz besonders bevorzugt wird als Referenz die noch nicht anvernetzte Referenzkautschukmischung eingesetzt.

In einer weiteren bevorzugten Ausführungsform wird die Signalstärke des Ultraschallwellensignals der Referenz durch die Extrusion einer Kautschukmischung von mindestens 1 kg bei vergleichbaren Prozess- und Messbedingungen während der Extrusion ermittelt. Vergleichbare Prozess- und Messbedingungen während der Extrusion und der Messung liegen dann vor, wenn die Änderungen des Durchsatzes kleiner als 10%, die Änderungen der Temperatur des Extrudats zwischen den Ultraschallwandlern und der Ultraschallwandler kleiner als $\pm 5$ K und Änderungen des Druckes p im Extrudat zwischen den Ultraschallwandlern kleiner als $\pm 5$ bar sind. Bevorzugt sind Prozess- und Messbedingungen, wenn die Änderungen des Durchsatzes kleiner als 5%, die Änderungen der Temperatur des Extrudates zwischen den Ultraschallwandlern und der Ultraschallwandler kleiner als $\pm$ 2 K und Änderungen des Druckes im Extrudat zwischen den Ultraschallwandlern kleiner als $\pm$ 2 bar sind. Besonders bevorzugt sind Prozess- und Messbedingungen, wenn die Änderungen des Durchsatzes kleiner als 2%, die Änderungen der Temperatur des Extrudates zwischen den Ultraschallwandlern und der Ultraschallwandler kleiner als $\pm$ 1 K und Änderungen des Druckes im Extrudat zwischen den Ultraschallwandlern kleiner als $\pm$ 1 bar sind.

In einer weiteren Ausführungsform der Erfindung werden die Werte des Ultraschallsignales quadriert und anschließend integriert und so die integrale Ultraschallwellenintensität I ermittelt. Alternativ kann die integrierte Ultraschallwellenamplitude A aus der Quadratwurzel der Ultraschallwellenintensität I ermittelt werden.

Besonders bevorzugt wird der relative Dämpfungskoeffizient $\alpha_{rel}$ gemäß der Formel (1b) mit Hilfe der integrierten Ultraschallwellenintensität I und bzw. der integrierten Ultraschallwellenamplitude A ermittelt.

$$\alpha_{rel} = (\ln A_{ref.} - \ln A)/x = (\ln I_{ref.} - \ln I)/2x \qquad (1b)$$

In einer bevorzugten Ausführungsform der Erfindung wird mit Hilfe der Fast Fourier Transformation (FFT) das Frequenzspektrum a(f) des Ultraschallwellenamplitude bzw. der Ultraschallwellenintensität i(f) ermittelt.

Bevorzugt wird der relative Dämpfungskoeffizient $\alpha_{rel}$ in Abhängigkeit der Frequenz f gemäß der Formel (1c)

$$\alpha_{rel}(f) = (\ln a_{ref.}(f) - \ln a(f))/x = (\ln i_{ref.}(f) - \ln i(f))/2x \qquad (1c)$$

aus der Ultraschallwellenamplitude a(f) der Ultraschallwellenamplitude $a_{ref.}(f)$ einer Referenz bzw. aus der Ultraschallwellenintensität i(f) der Ultraschallwellenintensität $i_{ref}(f)$. einer Referenz und dem Abstand x zwischen den Ultraschallwandlern ermittelt.

In einer besonders bevorzugten Ausführungsform der Erfindung wird das Frequenzspektrum in Frequenzbereiche mit einer minimalen Frequenz von $f_{min.}$ und einer maximalen Frequenz $f_{max}$ unterteilt und integriert. Bevorzugt enthalten diese Frequenzbereiche ein Maximum der Ultraschallwellenamplitude bzw. der Ultraschallwellenintensität.

Bevorzugt wird der relative Dämpfungskoeffizient $\alpha_{rel}$ in Abhängigkeit des Frequenzbereiches von einschließlich $f_{min.}$ bis einschließlich $f_{max.}$ gemäß der Formel (1d)

$$\alpha_{rel.}(f_{min.}, f_{max.}) = (\ln A_{ref.}(f_{min.}, f_{max.}) - \ln A(f_{min.}, f_{max.}))/x$$

$$= (\ln I_{ref.}(f_{min.}, f_{max.}) - \ln I(f_{min.}, f_{max.}))/2x \qquad (1d)$$

aus der Ultraschallwellenamplitude $A(f_{min.}, f_{max.})$, der Ultraschallwellenamplitude $A_{ref.}(f_{min.}, f_{max.})$ einer Referenz und dem Abstand x zwischen den Ultraschallwandlern ermittelt.

Alternativ kann der relative Dämpfungskoeffizient $\alpha_{rel}$ aus der Ultraschallwellenintensität $I(f_{min.}, f_{max.})$, Ultraschallwellenintensität $I_{ref.}(f_{min.}, f_{max.})$ einer Referenz und dem Abstand x gemäß der Formel (1d) ermittelt werden.

Die Standardabweichung $\sigma$ des relativen Dämpfungskoeffizienten $\alpha_{rel}$ wird nach den dem Fachmann bekannten Verfahren aus den Messwerten, bevorzugt während der Extrusion, für eine bestimmte Anzahl n von zeitlich aufeinanderfolgenden Messwerten ermittelt. Die Messzeit für die Standardabweichung ergibt sich aus der Anzahl der Messwerte n und dem zeitlich konstanten Abstand zwischen den Messwerten. Zunächst wird der Mittelwert $\overline{\alpha_{rel}}$ des relativen Dämpfungskoeffizienten in der Messzeit bestimmt entsprechend Formel (1e). Die Summe des Quadrates der Differenz der einzelnen Messwerte in der Messzeit zum Mittelwert, normiert auf die Gesamtzahl n der Messwerte, ist die Varianz $\sigma^2$ des relativen Dämpfungskoeffizienten in der Messzeit. Die Wurzel aus der Varianz ist die Standardabweichung $\sigma$ des relativen Dämpfungskoeffizienten $\alpha_{rel}$ (Formel (1f)).

$$\overline{\alpha_{rel}} = \sum_0^n \alpha_{rel}(n) \qquad (1e)$$

$$\sigma = \sqrt{\frac{\sum_0^n (\alpha_{rel}(n) - \overline{\alpha_{rel}})^2}{n}} \qquad (1f)$$

Bevorzugt wird der relative Dämpfungskoeffizient $\alpha_{rel}$ bei der Eigenfrequenz der Ultraschallwandler ermittelt.

In einer bevorzugten Ausführungsform wird der Abstand x zwischen den Ultraschallwandlern mit Hilfe eines Innentasters mit einer Genauigkeit von kleiner oder gleich 0,1 mm ermittelt.

Bevorzugt werden bei der Ermittlung die Standardabweichung $\sigma$ des relativen Dämpfungskoeffizienten $\alpha_{rel}$ weniger als < 1000 Messwerte, besonders bevorzugt weniger als 100 Messwerte, ganz besonders bevorzugt weniger als 20 Messwerte verwendet.

Bevorzugt wird die gleitende Standardabweichung $\sigma_g$ des relativen Dämpfungskoeffizienten $\alpha_{rel}$ ermittelt. Die gleitende Standardabweichung $\sigma_g$ berechnet sich aus dem gleitenden Mittelwert von $\alpha_{rel}$, berechnet nach Formel (1g), gemäß Formel (1h). Die Variablen x und y stellen in den Formeln (1g) und (1h) die obere bzw. die untere Grenze des Zeitfensters zur Bestimmung der gleitenden Standardabweichung $\sigma_g$ dar.

$$\overline{\alpha_g} = \sum_{n-y}^{n+x} \alpha_{rel}(n) \qquad (1g)$$

$$\sigma_g = \sqrt{\frac{\sum_{n-y}^{n+x}(\alpha_{rel}(n)-\overline{\alpha_g})^2}{n}} \qquad (1h)$$

In einer weiteren Ausführungsform wird neben dem relativen Dämpfungskoeffizienten die Schallgeschwindigkeit $V_S$ der Kautschukmischung gemäß Formel (2) ermittelt.

$$V_S = x/(t_{oF} - t_{US}) \qquad (2)$$

Die Zeitspanne $t_{oF}$ benötigt das Ultraschallsignal vom Zeitpunkt des Aussendens im Sender bis zum Empfangen im Empfänger. Die Zeitspanne $t_{US}$ benötigt das Ultraschallsignal in den Ultraschallwandlern. Die Zeitspanne $t_{US}$ wird bei vergleichbaren Temperaturen für Ultraschallwandler, bevorzugt wie während der Extrusion, ermittelt. Die Ermittlung der Zeitspanne $t_{US}$ erfolgt durch Bestimmung von $t_{oF}$ bei variierendem Abstand x der Ultraschallwandler. Dabei wird bevorzugt die Referenz zwischen den Ultraschallwandlern verwendet. Bevorzugt werden mindestens vier Abstände im Bereich von 5 mm bis 30 mm eingestellt. Die Zeitspanne $t_{US}$ ergibt sich aus dem Achsenabschnitt einer linearen Regression der Auftragung von $t_{oF}$ gegen den Abstand x der Ultraschallwandler.

Ungenügend silanisierte helle Füllstoffe führen zu einer Erniedrigung der Ultraschallwellenamplitude a bzw. der Ultraschallintensität i am Empfänger und einer Erhöhung des relativen Dämpfungskoeffizienten $\alpha_{rel}$ Die Schallgeschwindigkeit $V_s$ bleibt unverändert. Die Standardabweichung $\sigma$ des relativen Schalldämpfungskoeffizienten $\alpha_{rel}$ gibt die Homogenität der Kautschukmischung wieder.

In einer bevorzugten Ausführungsform durchläuft die Kautschukmischung ein Detektionsband aus einem oder mehreren Ultraschallsensorpaaren, die über die gesamte Breite des Fließkanals angeordnet sind.

In einer besonders bevorzugten Ausführungsform durchläuft die Kautschukmischung zwei Detektionsbänder, die leicht versetzt angeordnet sind. Der Vorteil hiervon ist, dass ein Großteil des Kautschukmischung, bevorzugt die komplette Kautschukmischung, analysiert wird.

**Vorrichtung**

Die Erfindung umfasst weiter auch eine Vorrichtung enthaltend eine Anordnung aus einem Extruder, mindestens einem Detektionsband aus mindestens 2 nebeneinander angeordneten Ultraschallsensorpaaren und mindestens eine Auswerteeinheit zur Durchführung des erfindungsgemäßen Verfahrens zur Prüfung der Silanisierung von hellen Füllstoffen. Bevorzugt ist das Detektionsband ein Bestandteil des Extruders.

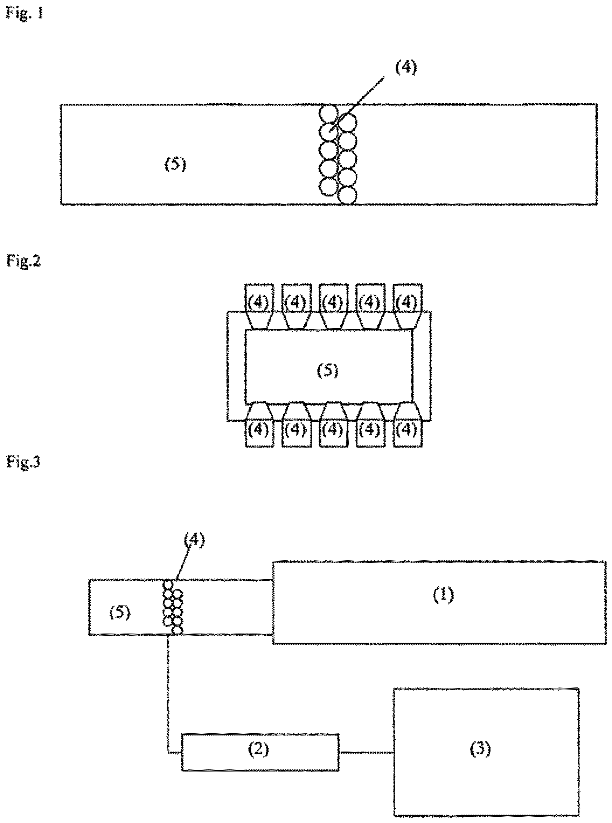

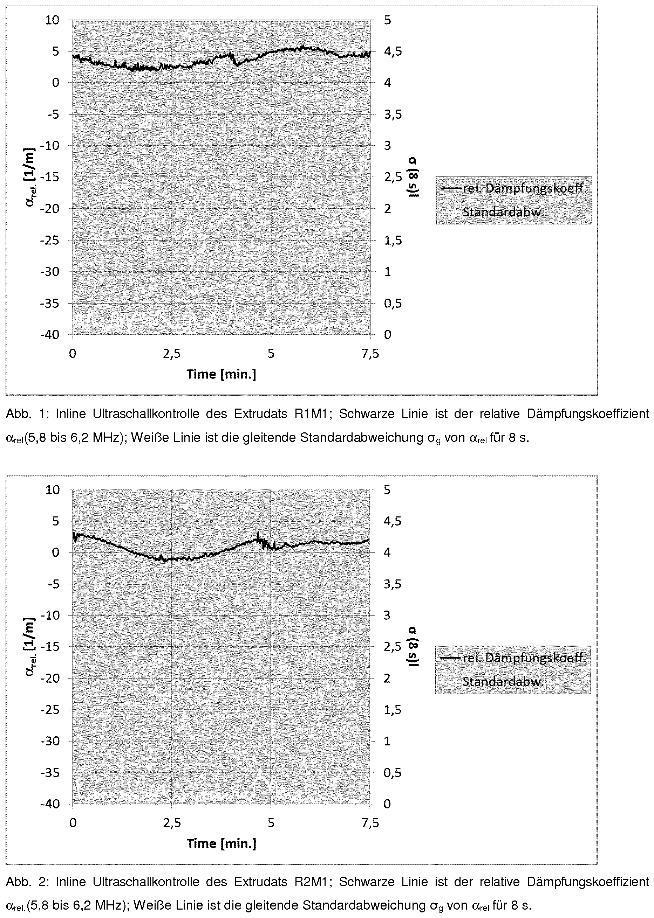

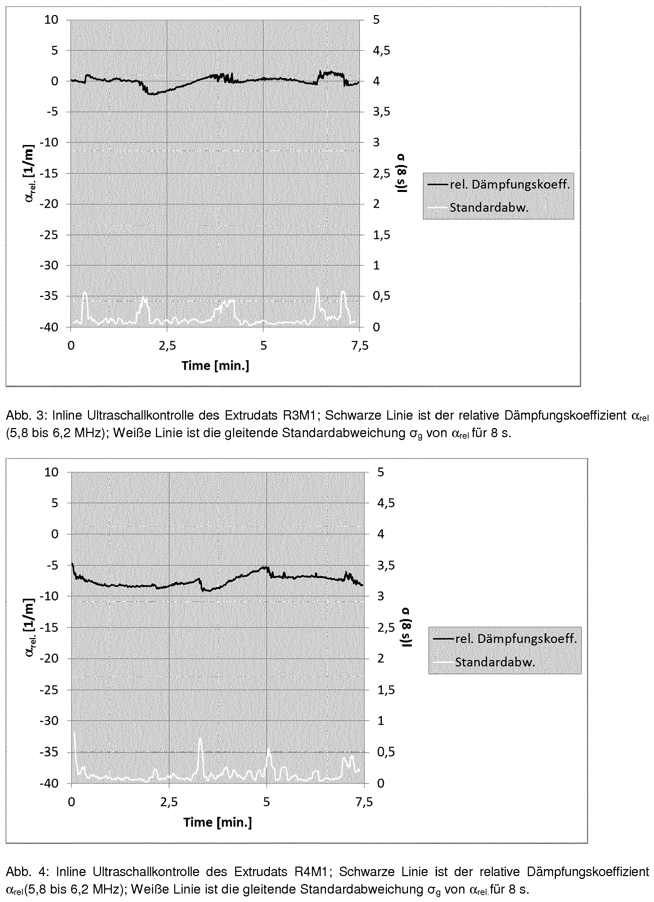

Bevorzugte Ausführungsformen der erfindungsgemäßen Vorrichtung sind in Fig.1 bis Fig. 3 dargestellt. Fig. 1 beschreibt exemplarisch das Detektionsband, bestehend aus den Ultraschallsensorpaaren (4), d.h. pro Paar jeweils ein Sender und ein Empfänger und dem Fließkanal (5) in Aufsicht. In Fig. 2 ist die Anordnung in Fig. 1 in Seitenansicht dargestellt. Fig. 3 zeigt eine Anordnung aus einem Extruder (1), dem Detektionsband, bestehend aus den Ultraschallwandlerpaaren (4) und dem Fließkanal (5) und der Ultraschallprüfelektronik (2) sowie einem Rechner als Auswerteeinheit (3).

**Verfahren zur Herstellung von einer mindestens einen silanisierten hellen Füllstoff enthaltenden Kautschukmischung**

Gegenstand der Erfindung ist zudem ein Verfahren zur Herstellung von einer mindestens einen silanisierten hellen Füllstoff enthaltenden Kautschukmischung, wobei mindestens ein heller Füllstoff mit mindestens einem Kautschuk und mindestens einem Silanisierungsmittel gemischt wird bei einer Temperatur von größer als 160°C und wobei zumindest ein Teil der erhaltenen Kautschukmischung durch das erfindungsgemäße Verfahren zur Prüfung der Silanisierung geprüft wird.

Vorzugsweise werden mehr als 1 Vol.-%, besonders bevorzugt mehr als 10 Vol.-%, ganz besonders bevorzugt mehr als 25 Vol.-%, ganz ganz besonders bevorzugt mehr als 50 Vol.-%, meist bevorzugt mehr als 80 Vol.-% der erhaltenen Kautschukmischung durch das erfindungsgemäße Verfahren zur Prüfung der Silanisierung geprüft.

Bevorzugt erfolgt das Verfahren zur Herstellung bei einer Temperatur von größer als 160°C bis 200°C, besonders bevorzugt von größer als 160°C bis 180°C.

Bevorzugt ist das obige Verfahren ein Verfahren zur Herstellung von einer mindestens einen silanisierten hellen Füllstoff enthaltenden Kautschukmischung, wobei mindestens ein heller Füllstoff mit mindestens einem Kautschuk und mindestens einem Silanisierungsmittel gemischt wird bei einer Temperatur von größer als 160°C und wobei zumindest ein Teil der erhaltenen Kautschukmischung durch das erfindungsgemäße Verfahren zur Prüfung der Silanisierung geprüft wird und wobei die gleitende Standardabweichung $\sigma_g$ des relativen Dämpfungskoeffizienten $\alpha_{rel}$ der Kautschukmischung kleiner als 0,5 [1/m] ist, bevorzugt kleiner als 0,3 [1/m], bezogen auf eine Messzeit von 1 bis 100 s, bevorzugt von 1 bis 50 s, besonders bevorzugt 1 bis 15 s, und der relative Dämpfungskoeffizient $\alpha_{rel}$ der Kautschukmischung kleiner als 0 [1/m] ist, bevorzugt kleiner als -5 [1/m], besonders bevorzugt kleiner als -5 [1/m] und gleich oder größer als -15 [1/m] ist.

Die nach dem zuvor genannten bevorzugten Verfahren erhaltene Kautschukmischung liegt bevorzugt noch nicht anvernetzt vor.

Das erfindungsgemäße Verfahren kann dabei kontinuierlich oder als Batchverfahren betrieben werden.

In einer bevorzugten Ausführungsform wird das Verfahren zur Herstellung von einer mindestens einen silanisierten hellen Füllstoff enthaltenden Kautschukmischung vor der Extrusion der erhaltenen Kautschukmischung durchgeführt, und das Verfahren zur Prüfung der Silanisierung erfolgt durch Beschallung des durch die Extrusion erhaltenen Extrudats in einem Extruder.

In einer weiteren bevorzugten Ausführungsform werden das Verfahren zur Herstellung von einer mindestens einen silanisierten hellen Füllstoff enthaltenden Kautschukmischung und das erfindungsgemäße Verfahren zur Prüfung der Silanisierung in demselben Extruder durchgeführt. Hierbei werden mindestens ein heller Füllstoff, mindestens ein Silanisierungsmittel und mindestens ein Kautschuk dem Extruder zugeführt und in diesem aus dem Silanisierungsmittel und dem hellen Füllstoff der silanisierte helle Füllstoff gebildet.

Optional können in der zuletzt genannten Ausführungsform mindestens ein heller Füllstoff, mindestens ein Kautschuk und mindestens ein Silanisierungsmittel bei einer Temperatur von kleiner oder gleich 160°C innerhalb oder außerhalb des Extruders vorgemischt werden, und diese Mischung dann in dem Extruder beschallt werden.

Für die in dem Verfahren zur Herstellung von einer mindestens einen silanisierten hellen Füllstoff enthaltenden Kautschukmischung eingesetzten Komponenten, beinhaltend aber nicht beschränkt auf den mindestens einen hellen Füllstoff, den mindestens einen Kautschuk und das mindestens eine Silanisierungsmittel, gelten die bezüglich des erfindungsgemäßen Verfahrens zur Prüfung der Silanisierung weiter oben angeführten Beschreibungen und Vorzugsbereiche analog.

In dem Verfahren zur Herstellung von einer mindestens einen silanisierten hellen Füllstoff enthaltenden Kautschukmischung werden bevorzugt 5 bis 15 Gew.-% mindestens eines Silanierungsmittels, bezogen auf den Gesamtanteil an hellen Füllstoffen in der Kautschukmischung, eingesetzt.

**Weitere Zusatzstoffe**

Die mindestens einen silanisierten hellen Füllstoff enthaltenden Kautschukmischungen können weitere Zusatzstoffe wie weitere Füllstoffe, weitere Additive wie Alterungsschutzmittel, Weichmacher, weitere Vernetzungsmittel, Vulkanisationsbeschleuniger und/oder Vulkanisationsverzögerer und/oder weitere Hilfsmittel enthalten.

Als weitere Füllstoffe werden im Sinne der vorliegenden Erfindung andere als der weiter oben näher beschriebene mindestens eine helle Füllstoff und der mindestens eine silanisierte helle Füllstoff verstanden.

Weitere Füllstoffe sind z.B. Kohlenstoff-basierte Füllstoffe, wie z.B. Ruß, Graphit, Carbon Nanotubes, sowie magnetisierbare Füllstoffe wie Carbonyl-Eisenpulver, Eisenoxide, Ferrite und/oder Fasern wie z.B. Aramidfaserpulpe und Kohlefasern.

Als Alterungsschutzmittel sind verfärbende und nicht verfärbende Alterungsschutzmittel, wie z.B. Paraphenylendiamine, Isopropylphenylparaphenylendiamin (IPPD), Para-Phenylen-Diamin (6PPD), N,N-ditoly-p-phenylendiamin (DTPD), Amine, wie z.B. Trimethyl-1,2-dihydrochinolin (TMQ), (Phenyl)amine]-1,4-naphthalendion (PAN), Bis(4-octylphenyl)amin (ODPA), styrolisiertes Diphenylamin (SDPA), Mono- und Bisphenole, wie z.B. 2,2'-Methylen-bis-(4-methyl-6-tert.butylphenol (BPH), 2,2'-Isobutyliden-bis-(4,6-dimethyl-phenol) (NKF), 2,2'-Dicyclo-pentadienyl-bis-(4-methyl-6-tert.-butyl-phenol) (SKF), 2,2'-Methylen-bis-(4-methyl-6-cyclohexyl-phenol (ZKF), 2,6-Di-tert.-butyl-p-kresol (BHT), substituiertes Phenol (DS), styrolisierte Phenole (SPH), Mercatobenzimidazole, wie z.B. 2-Mercaptobenzimidazol (MBI), 2-Mercaptomethyl-benzimidazole (MMBI), Zink-4- und -5-methyl-2-mercapto-benzimidazole (ZMMBI) geeignet.

Weichmacher sind z.B. langkettige Ester und/oder Ether, wie Thioester, Phthalsäureseter, Alkylsulfonsäureester, Adipinsäureester, Sebacinsäuresester, Dibenzylether, und/oder Mineralöle (paraffinische, aromatische naphthenische oder synthetische Öle).

Als weitere Vernetzungsmittel werden im Sinne der vorliegenden Erfindung andere als der weiter oben näher beschriebene mindestens eine silanisierte helle Füllstoff und das mindestens eine Silanisierungsmittel verstanden.

Weitere Vernetzungsmittel können sein:

Netzknotenbildner: Netzknotenbildner sind Moleküle, die zwei einzelne Polymerketten mit einander verbinden können, wie z.B.

- Schwefel (löslich oder unlöslich) und/oder Schwefelspender, wie z.B. Dithiomorpholine (DTDM), Tetramethylthiuramdisulphide (TMTD), Tetraethylthiuramdisulphid (TETD), Dipentamethylenthiuramterasulphide (DPTT), Phosphorylpolysulfide, wie z.B. Rhenocure® SDT/S der Firma Rhein Chemie Rheinau GmbH und/oder
- Peroxide, wie z.B. Di-tert.Butyl-Peroxide, Di-(tert,Butyl-Peroxy-Timethyl-Cyclohexane, Di-(tert.-Butyl-peroxy-isopropyl)benzene, Dicumylperoxide, Dimethyl-di(tert.butyl-Peroxy)hexin, Butyl-Di-(tert,Butyl-Peroxy-)valerat,
- Resorcinol, Aldehyd-Amin-Kondensationsprodukte, wie z.B. Hexamethylentetramin, Resorcinol-Formaldehyd-Vorkondensate und/oder Vulkanisationsharze, wie zum Beispiel Halomethylphenolharz,
- Chinondioxime
- Bisphenole.

Vulkanisationsbeschleuniger sind beispielsweise:

- Carbamate bzw. Triazine, wie z.B. Hexamethylendiamincarbamat (HMDC), organische Triazine,
- Thiazole, wie z.B. 2-Mercaptobenzothiazol (MBT), Zinkmercaptobenzothiazol (ZnMBT), Thiadiazole (TDD),
- Sulfenamide, wie Cyclohexyl-benzothiazol-Sulphenamide (CBS), Di-benzothiazyl-disulphid (MBTS), Butyl-benzothiazole-Sulphenamide (TBBS), Dicyclohexyl-Benzothiazol-Sulphenamid (DCBS), 2-(4-Morpholinylmercapto)-benzothiazol (MBS),
- Thiurame, wie Tetramethyl-thiuram-monosulphid (TMTM), Tetraethyl-thiuram-disulphid (TETD), Tetramethyl-thiuram-disulphid (TMTD), Tetrabenzylthiuram Disulphid (TBTD), Dipentamethylene-Thiuram-Tetra(Hexa)-Sulphid (DPTT),
- Dithiocarbamate, wie Zn-Dimethyldithiocarbamate (ZDMC), Cu-Dimethyldithiocarbamate, Bi- Dimethyldithio-carbamate, Zn-Diethyldithiocarbamate (ZDEC), Tellurdiethyldithio-carbamate (TDEC), Zn-Dibuthyldithiocarbamate (ZDBC), Zn-Ethyl-Phenyl-Dithiocarbamate (ZEPC), Zn-Dibenzyl-Dithiocarbamate (ZBEC), Ni-Dibuthyl-Dithiocarbamate (NBC), Selendiethyldithiocarbamate (SeEDC), Selendimethyldithiocarbamate (SeDMC), Tellurdiethyldithiocarbamate (TeEDC),
- Thiophosphat- und Dithiophosphat, wie z.B. Zink-O,O-di-n-butyl-dithiophosphat (ZBDP), Zink-O-butyl-O-hexyl-dithiophosphat, Zink-O,O-diisooctyl-dithiophosphat (ZOPD), Dodecylammonium-diisooctyl-dithiophosphat (AOPD), wie z.B. die Rhenogran®-Typen ZDT, ZAT, ZBOP der Firma Rhein Chemie Rheinau GmbH,
- Harnstoff/Thioharnstoffe, wie z.B. Ethylenthioharnstoff (ETU), N,N,N'N'-Tetramethyl-thioharnstoff (TMTU), Diethylthioharnstoff (DETU), Dibutylthioharnstoff (DBTU), 3-(3,4-Dichlorphenyl)-1,1-dimethylharnstoff (Diuron) etc., und/oder
- Xanthogenatbeschleuniger, wie z.B. Zinkisopropylxanthogenat (ZIX),
- Guanidine, wie z.B. Diphenylguanidin (DPG) und/oder N',N-di-ortho-tolyl-Guanidin (DOTG) und die Guanidine-frei Ersatzbeschleuniger, wie Rhenogran ® XLA 60,

Vulkanisationsverzögerer sind z.B. N-Nitrososdiphenylamin, N-Cylcohexylthiophthalimid (CPT), z.B. Vulkalent® G), Sulfonamidderivate (z.B. Vulkalent® E/C), Phthalsäureanhydrid (z.B. Vulkalent® B/C), oder Benzoesäureanhydrid.

Hilfsmittel sind z.B. Dispergierhilfsmittel, wie z.B. Fettsäuren, Stearinsäure, Ölsäure, Aktivatoren wie zum Beispiel Zinkoxid, Bleioxid, Bismuthoxid, Lithiumcarbonat, Natriumcarbonat und/oder Calciumhydroxid, Magnesiumoxid, Flammschutzmittel wie Antimonoxid, etc.

Die vorgenannten weiteren Füllstoffe, Additive, Weichmacher, weiteren Vernetzungsmittel, Vulkanisationssechleuniger und/oder Vulkanisationsverzögerer, Hilfsmittel usw. sind dem Fachmann vertraut und können auch in granulierter Form, z.B. als polymergebundene Additive oder in Form der in EP2314442A beschriebenen Vernetzerbatches eingesetzt werden.

Die Kautschukmischungen können weiterverarbeitet werden, beispielsweise zu Masterbatches, Grundmischungen und vernetzbaren Kautschukmischungen.

Masterbatches haben typischerweise einen hohen Anteil an Additiven bezogen auf Kautschuk und enthalten beispielsweise:

2,5 bis 90 Gew.-% Kautschuk
0 bis 50 Gew.-% Weichmacher
2 bis 80 Gew.-% silanisierte helle Füllstoffe
0 bis 20 Gew.-% Dispergierhilfsmittel
0 bis 10 Gew.-% Silan

Grundmischungen sind Kautschumischungen, denen vor der praktischen Anwendung noch Vernetzungsmittel und ggf. Vulkanisationsbeschleuniger zugesetzt werden müssen, beispielsweise:

100 phr Kautschuk
0 bis 100 phr Weichmacher
0 bis 200 phr Füllstoffe, davon mindestens 10% silanisierte helle Füllstoffe
0 bis 30 phr Hilfsmittel und
0 bis 10 phr Alterungsschutzmittel.

Vernetzbare Kautschukmischungen sind Kautschukmischungen, die zusätzlich 0,01 bis 20 phr ein oder mehrere weitere Vernetzungsmittel und optional Vulkanisationsbeschleuniger und optional Vulkanisationsverzögerer enthalten.
Besonders bevorzugt sind vernetzbare Kautschukmischungen, die 30 bis 110 phr Ruß und gefällte Kieselsäure, 3 bis 9 phr Silan, 2 bis 7 phr Zinkoxid sowie 0,5 phr bis 4 phr Schwefel und 1 bis 5 phr Beschleuniger enthalten.
Die Erfindung umfasst auch ein Verfahren zur Herstellung von vernetzbaren, mindestens einen silanisierten hellen Füllstoff enthaltenden Kautschukmischungen, in dem nach der Herstellung der mindestens einen silanisierten hellen Füllstoff enthaltenden Kautschukmischung ein oder mehrere weitere Vernetzungsmittel zugefügt werden.
Die Herstellung der Masterbatches, der Grundmischung und der vernetzbaren Kautschukmischung erfolgt vorzugsweise nach den dem Fachmann geläufigen Verfahren, wie beispielsweise beschrieben in PCT/EP2009/058041. Füllstoffe, Hilfsmittel, weitere Vernetzungsmittel und/oder Alterungsschutzmittel werden dabei zusammen mit dem Kautschuk in einem Mischaggregat vermischt. Geeignete Mischaggregate sind zum Beispiel Innenmischer, Walzwerke und Extruder. Besonders geeignet sind Innenmischer mit Austragsextruder.
In einer Ausführungsform der Erfindung wird in einem ersten Schritt die mindestens einen silanisierten hellen Füllstoff enthaltende Kautschukmischung nach dem erfindungsgemäßen Verfahren hergestellt. In einem folgenden Schritt wird die Mischung homogenisiert.
In einer weiteren Ausführungsform der Erfindung wird in einem ersten Schritt die mindestens einen silanisierten hellen Füllstoff enthaltende Kautschukmischung nach dem erfindungsgemäßen Verfahren hergestellt. In einem folgenden Schritt wird die Mischung homogenisiert. In einem folgenden Schritt werden nach Abkühlen der Kautschukmischung auf eine Temperatur kleiner oder gleich 130°C ein oder mehrere weitere Vernetzungsmittel in einem weiteren Mischaggregat hinzugegeben. Diese Ausführungsform findet bevorzugt Anwendung bei der großtechnischen Herstellung der mindestens einen silanisierten hellen Füllstoff enthaltenden Kautschukmischung, besonders bevorzugt der der Herstellung von mehr als 100 kg.
"Folgender Schritt" bedeutet in diesem Zusammenhang, dass der entsprechende Schritt auf den zuvor genannten Schritt folgen muss, allerdings nicht unmittelbar. Es können zwischen dem zuvor genannten Schritt und dem folgenden Schritt auch noch ein oder mehrere weitere Schritte liegen.
Besonders bevorzugt sind dabei kontinuierliche Verfahren, bei denen die Masterbatche, Grundmischungen oder auch die vernetzbaren Kautschukmischungen in einem oder mehreren Prozessschritten mit einem oder mehreren Extrudern hergestellt werden.
Ganz besonders bevorzugt ist das in DE-A-102008040138 beschriebene Herstellungsverfahren für vernetzbare Kautschukmischungen, in dem die Grundmischung in mindestens einem diskontinuierlichen Kneterprozess hergestellt wird, und weitere Vernetzungsmittel in Form der in beschriebenen EP-A-2314442 beschriebenen Vernetzerbatche zur Grundmischung hinzugefügt werden und die Vernetzerbatche in einem kontinuierlichen Prozess mit einem Extruder mit der Grundmischung vermischt werden.
Weiterhin betrifft die Erfindung ein Verfahren zur Herstellung von Vulkanisaten umfassend die erfindungsgemäße Herstellung von vernetzbaren, silanisierte helle Füllstoffe enthaltenden Kautschukmischungen und die nachfolgende Vulkanisation bei einer Temperatur im Bereich von 100°C bis 200°C.
Ebenfalls mitumfasst von der Erfindung ist die Verwendung von Messvorrichtungen zur Bestimmung der Ultraschallwellenamplitude a, integrierten Ultraschallwellenamplitude A und/oder der Ultraschallwellenintensität i, der integrierten Ultraschallwellenintensität I einer oder mehreren Frequenzen in einem Bereich von 4 bis 10 MHz, vorzugsweise von 5 bis 7 MHz, für das erfindungsgemäße Prüfverfahren, für die erfindungsgemäßen Herstellungsverfahren von Kautschukmischungen im allgemeinen und vernetzbaren Kautschukmischungen im speziellen. Vorzugsweise umfasst dies auch die Verwendung der genannten Messvorrichtungen zur Bestimmung des relativen Dämpfungskoeffizienten $\alpha_{rel}$ bei den oben genannten Verfahren.

Somit stellt die vorliegende Erfindung ein Verfahren zur Prüfung der Silanisierung zur Verfügung, welches eine inline Kontrolle der in situ Silanisierung von hellen Füllstoffen, insbesondere gefällten Kieselsäuren, erlaubt. Das erfindungsgemäße Verfahren ermöglicht eine kostengünstige Herstellung von silanisierten hellen Füllstoffen enthaltenden Kautschukmischungen, da die Herstellung bei hohen Temperaturen, insbesondere von größer als 160°C, erfolgen kann. Der relative Dämpfungskoeffizient $\alpha_{rel}$ der Kautschukmischung sowie die Standardabweichung $\sigma$ des relativen Dämpfungskoeffizienten $\alpha_{rel}$, bevorzugt die gleitende Standardabweichung $\sigma_g$, dienen zur Detektion des Anvernetzens der Kautschukmischung.

Das Verfahren arbeitet zudem zerstörungsfrei und weist eine hohe Toleranz für Ruß als zusätzlichem Bestandteil der Kautschukmischung auf.

Die in der Beschreibung verwendeten Überschriften sollen lediglich der Gliederung dienen, ohne dabei limitierend zu wirken.

Die nachfolgenden Beispiele, Abbildungen und Figuren dienen der Erläuterung der Erfindung, ohne dabei limitierend zu wirken.

**Beispiele:**

Die Komponenten einer typischen Kautschukmischung für Reifenlaufflächen gemäß Tabelle 1 wurden bereitgestellt.

Tabelle 1: Rezeptur einer typischen Kautschukmischung für Reifenlaufflächen

| Handelsname | Inhaltsstoffe | | Dichte [g/cm$^3$] | Menge [phr] |
|---|---|---|---|---|
| Buna® CB 24 | Butadienkautschuk | Lanxess Deutschland GmbH | 0,91 | 15 |
| Buna® VSL 5025-2 HM | Lösungs-Styrol-Butadien-Kautschuk | Lanxess Deutschland GmbH | 0,95 | 116,9 |
| Corax® N-234 | Ruß | Orion Engineered Carbons GmbH | 1,8 | 35 |
| Ultrasil® VN3 | Kieselsäure | Evonik Industries AG | 2 | 55 |
| ASM-TMQ | Alterungsschutzmittel 2,2,4-Trimethyl-1,2-dihydrochinolin | Lanxess Deutschland GmbH | 1,1 | 3,0 |
| ASM-6PPD | Alterungsschutzmittel N-1,3-Dimethylbutyl-N'-phenyl-p-phenylendiamin | Lanxess Deutschland GmbH | 1 | 2,5 |
| Antilux® 654 | Alterungsschutzmittel Gemisch ausgewählter Paraffine und Mikrowachse mit mittlerer Molekulargewichtsverteilung | Lanxess Deutschland GmbH | 0,9 | 2,0 |
| Aflux® 37 | Mischung oberflächenaktiver Substanzen mit Fettsäuren | Lanxess Deutschland GmbH | 0,98 | 2,0 |
| | Stearinsäure | Peter Greven GmbH & Co. KG | 0,89 | 1 |
| Weißsiegel | Zinkoxid | Norzinco GmbH | 5,6 | 2 |

(fortgesetzt)

| Handelsname | Inhaltsstoffe | | Dichte [g/cm³] | Menge [phr] |
|---|---|---|---|---|
| Si69 | Bis-(triethoxy-silyl-propyl)-tetrasulfid | Evonik Industries AG | 0,866 | 4,8 |

Diese Komponenten wurden gemäß der Mischvorschrift in Tabelle 2 in verschiedenen Chargen gemischt.

Tabelle 2: Mischvorschrift

| Zeit | Mischungsbestandteil | Bemerkung |
|---|---|---|
| 0-0,75' | Polymere | Stempel unten |
| 0,75'-1'20" | Ruß, Kieselsäure + Si69 + ZnO | Stempel oben |
| 1'20" - | | Stempel ab |
| 2' | Alterungsschutzmittel, Stearinsäure, Aflux® 37 | |
| 3,5' -4 | | Kehren Stempel oben |
| 4'-5' | | Stempel unten |
| 5' | | Auswurf |

Bei den verschiedenen Chargen R1M1 bis R5M1 wurde der Mischparameter Drehzahl variiert gemäß Tabelle 3. Dabei stehen R1-R5 für die verschiedenen Rezepturen und M1 für die Mischstufe 1.

Tabelle 3: Prozessparameter des Innenmischers zur in situ Silanisierung einer typischen Kautschukmischung aus Tabelle 1

| Batch | | R1M1 | R2M1 | R3M1 | R4M1 | R5M1 |
|---|---|---|---|---|---|---|
| **Mischparameter** | | | | | | |
| Füllgrad | [%] | 70 | 70 | 70 | 70 | 70 |
| Drehzahl | [U/min.] | 40 | 50 | 70 | 100 | 135 |
| Vorlauftemperatur | [°C] | 70 | 70 | 70 | 70 | 70 |
| Mischzeit | [min.] | 5 | 5 | 5 | 5 | 5 |
| Auswurftemperatur $T_A$ | [°C] | 120 | 135 | 150 | 165 | 180 |

Die Mischungen R1M1- R5M1 werden im 1,5 l Innenmischer (Intermesh) der Firma Gumix S.A. für 5 min gemischt.

Durch die unterschiedliche Drehzahl des Innenmischers werden unterschiedliche Werte der Mischungstemperatur erreicht. Die Auswurftemperatur $T_A$, gemessen nach einem dem Fachmann bekannten Verfahren mit einem Einstichthermometer unmittelbar nach Auswurf der Kautschukmischung aus dem Innenmischer, ist ein Maß für die Mischungstemperatur im Innenmischer und damit für die Temperatur, bei der die Kautschukmischung hergestellt wird.

Tabelle 3 gibt die Auswurftemperatur $T_A$ der Kautschukmischungen mit gleicher Zusammensetzung in Abhängigkeit der Prozessparameter zur in situ Silanisierung an. Die Auswurftemperatur $T_A$ der Mischungen R1M1 sowie R2M1 liegen deutlich unterhalb von 160°C. R3M1 hat eine Auswurftemperatur von 150°C und ist die Referenzkautschukmischung. Die Mischungen R4M1 und R5M1 wurden oberhalb von 160°C hergestellt.

Das Verfahren entspricht einem typischen Verfahren für in situ Silanisierungen in der Reifenindustrie für die erste Mischstufe.

Nach dem Mischprozess im Innenmischer wurden die Mischungschargen auf einer Doppelwalze Firma Rubicon Gummitechnik und Maschinentechnik GmbH bei einer Temperierung von 50°C ausgewalzt (dreimal links und dreimal rechts eingeschnitten) und zu einem Mischungsfell ausgezogen. Aus dem Fell wurden Streifen geschnitten. Diese Streifen wurden zu einem Einwellenextruder EEK32.12L der Firma Rubicon Gummitechnik und Maschinentechnik GmbH mit geringer Mischwirkung gefüttert. Am Ausgang des Extruders befindet sich ein Sensorkopf, ausgestattet mit zwei gegenüberliegenden Ultraschallwandlern mit einem Abstand von 10 mm, zu dem ein Temperatursensor T1 mit einem Schwert das in den Mischungsfluss hinein ragt, und ein Druck-/Temperatur-Sensor. Dieser Temperatursensor misst den Druck p und die Temperatur T2 an der Kautschuk-Metall-Grenzfläche. Der Fließkanal im Sensorkopf hat eine Breite von 20 mm und eine Höhe von 10 mm. Der Durchmesser der Ultraschallwandler beträgt 8 mm. Sensorkopf und Extruder wurden bei 100°C temperiert. Die Schneckendrehzahl betrug 20 U/min. Dies entspricht einem Durchsatz von ca. 1 kg Kautschukmischung pro 10 min.

Die inline Prüfung der Silanisierung wurde mit einem Paar von Ultraschallwandlern K6V1 der Firma GE Sensing & Inspection Technologies GmbH in Transmission durchgeführt. Die Ultraschallwandler wurden mit der Prüfelektronik PCM 100LAN der Firma Inoson GmbH angesteuert. Die Ultraschallpulse wurden alle 150 ms mit Hilfe des piezoelektrischen Effektes im Ultraschallwandler erzeugt. Der Ultraschallwandler wandelt die Spannungspulse in Ultraschallsignale um. Dabei wurden die Ultraschallwandler mit 7 Bursts bei der Eigenfrequenz der Ultraschallwandler bei 6 MHz angeregt. Der Ultraschallpuls des Transmitters wandert durch die extrudierende Kautschukmischung und wird dabei durch die Kautschukmischung gedämpft. Die Schwingungsamplitude a des Ultraschallsignales wird erniedrigt. Der Receiver empfängt das Schallsignal und wandelt es in ein Spannungssignal um. Das Spannungssignal wird von der Hardware des PCM100LAN von 35 bis 40 dB verstärkt und zu einem Rechner geleitet. Mit Hilfe des Rechners wird der erste Schallpuls des sogenannten A-Scans mit der sogenannten Fast Fourier Transformation (FFT) ausgewertet. Das so erhaltene Amplitudenspektrum a(f) in Abhängigkeit der Ultraschallfrequenz f wurde zwischen 5,8 und 6,2 MHz (vgl. Tabelle 4) integriert und logarithmiert. Die so erhaltenen Werte ln $A(f_{min.},f_{max.})$ für das logarithmierte Integral wurden für die verschiedenen Mischungschargen R1M1 bis R5M1 in Abhängigkeit der Zeit t für 7,5 min zusammen mit den Werten der Temperatursensoren T1 und T2 sowie des Druckes aufgezeichnet. Zusätzlich wurde die Zeit $t_{oF}$ ermittelt, die das Schallsignal zur Transmission vom Piezokristall des Emitters bis zum Piezokristall des Empfängers benötigt. Dies geschieht noch vor Austritt der Mischung aus dem Sensorkopf am Ausgang des Extruders. Die Verweilzeit der Mischung im Sensorkopf betrug weniger als 1 Minute.

Aus den Werten für ln $A(f_{min.},f_{max.})$ wird der relative Dämpfungskoeffizient $\alpha_{rel}(f_{min.},f_{max.})$ nach dem Lambert Beer'schen Gesetz berechnet.

$$\alpha_{rel}(f_{min.},f_{max.}) = (\ln A_{ref.}(f_{min.},f_{max.}) - \ln A(f_{min.},f_{max.})/x \quad (1d)$$

Hierbei ist x der Abstand zwischen den Ultraschallwandlern. In Formel (1d) ist der Mittelwert von ln $A_{ref.}(f_{min.},f_{max.})$ = 4,06 a.u. des Referenzextrudats R3M1 (mit einer Mischungstemperatur zwischen 140°C und 160°C) für eine Zeitspanne für die Extrusion von $\Delta t$ = 7,5 min. Der Abstand zwischen den Schallwandlern betrug 10 mm.

Das Ergebnis für den Frequenzbereich $f_{min.}$ = 5,8 MHz bis $f_{max.}$ = 6,2 MHz ist für jedes Extrudat in den Abb. 1 bis Abb. 5 dargestellt.

In Tabelle 4 sind die Mittelwerte für den relativen Dämpfungskoeffizient $\alpha_{rel}(f_{min.},f_{max.})$ für eine Zeitspanne von $\Delta t$ = 7,5 min für die Extrusion für jede Mischungscharge dargestellt, entsprechend den Abb. 1 bis 5. Diese Mittelwerte wurden berechnet nach Formel (1e). Zudem wurde jeweils der Mittelwert der Standardabweichung $\sigma$ des relativen Dämpfungskoeffizienten $\alpha_{rel}(f_{min.},f_{max})$ für $\Delta t$ = 7,5 min gemäß Formel (1f) ermittelt.

In Tabelle 4 ist auch die gleitende Standardabweichung $\sigma_g$ über einen Zeitraum von 8 s angegeben. Die gleitende Standardabweichung $\sigma_g$ berechnet sich aus dem gleitenden Mittelwert von $\alpha_{rel}$, berechnet nach Formel (1g), gemäß Formel (1h).

In den Abb. 1 bis Abb. 5 ist die Standardabweichung $\sigma_g$ für jeden Messpunkt n des zum Zeitpunkt t(n) für ein Zeitfenster von 51 Messpunkten (x = y = 25) von t(n-25) bis t(n+25) gleitend dargestellt. Der Buchstabe n bezeichnet hierbei die Nummer des Messpunktes. Es werden 25 Messwerte vor dem Zeitpunkt t(n), der Messwert zum Zeitpunkt t(n) und die 25 Messwerte nach dem Zeitpunkt t(n) zur Ermittlung der Standardabweichung $\sigma_g$ verwendet. Gleitend bedeutet, dass die Standardabweichung $\sigma_g$ für den darauffolgenden Messpunkt n+1 zum Zeitpunkt t(n+1) im Zeitfenster t(n-24) bis t(n+26) ermittelt wird. Das Zeitfenster aus 51 Messpunkten zur Ermittlung von $\sigma_g$ wird also um einen Messpunkt verschoben. Die gleitende Standardabweichung $\sigma_g$ von $\alpha_{rel}(f_{min.},f_{max.})$ wurde in den Abbildungen 4 bis 8 in einem Zeitfenster von 8 s ermittelt.

Außerdem ist der Mittelwert des Drucksensors p und die Mittelwerte für der Temperatursensor T1 mit Schwert und T2 ohne Schwert angegeben. Die Schallgeschwindigkeit $V_S$ wird aus dem Abstand x und der Zeit $t_{oF}$ bis zur ersten Spannungsamplitude ermittelt. Dabei muss die Zeit $t_{US}$ = 4,6 $\mu s$ des Ultraschallschallsignales im Vorlauf beider Ultraschallwandler berücksichtigt werden nach Formel (2).

$$V_S = x/(t_{oF} - t_{US}) \quad (2)$$

Zusätzlich zu den Ultraschallkennwerten wurde die Viskosität nach Mooney ML1+4 (100°C) ermittelt.

Tabelle 4: Ergebnisse der Messungen der extrudierenden Kautschukmischungen (Messbedingungen und Ultraschallkennwerte) sowie der extrudierten Kautschukmischungen (Mooney Viskosität und RPA 2000) aus Tabelle 3. Die Standardabweichung vom Mittelwert wird hinter dem Mittelwert mit "$\pm$" angegeben.

| Batch | Einheit | R1M1 | R2M1 | R3M1 | R4M1 | R5M1 |
|---|---|---|---|---|---|---|
| **Standard Prozesskontrolle** | | | | | | |
| Mooney Viskosität ML1+4 (100°C) | [MU] | 106 | 101 | 99 | 97 | 112 |
| RPA 2000 G'(1%, 1 Hz, 60°C) | [kPa] | 1145 | 1070 | 1032 | 941 | 876 |
| **Messbedingungen** | | | | | | |
| Druck p | [bar] | 72 $\pm$ 1 | 69 $\pm$ 1 | 68 $\pm$ 1 | 68 $\pm$ 1 | 74 $\pm$ 2 |
| T1 | [°C] | 95 $\pm$ 1 | 95 $\pm$ 1 | 95 $\pm$ 1 | 95 $\pm$ 1 | 95 $\pm$ 1 |
| T2 | [°C] | 104 $\pm$ 1 | 103 $\pm$ 1 | 103 $\pm$ 1 | 103 $\pm$ 1 | 103 $\pm$ 1 |
| **Ultraschallkennwerte** | | | | | | |
| $V_S$ | [m/s] | 1331 $\pm$ 1 | 1329 $\pm$ 1 | 1328 $\pm$ 1 | 1326 $\pm$ 2 | 1331 $\pm$ 2 |
| Relative Dämpfungskoeffizient $\alpha_{rel}$ (5,8 MHz bis 6,2 MHz) | [1/m] | 4 $\pm$ 1 | 1 $\pm$ 1 | 0 $\pm$ 1 | -8 $\pm$ 1 | -22 $\pm$ 3 |
| Gleitende Standardab weichung $\sigma_g$ von $\alpha_{rel}$ für 8 s | [1/m] | 0,2 | 0,1 | 0,2 | 0,1 | 1,3 |

Tabelle 4 zeigt, dass für Kautschukmischungen mit zunehmender Auswurftemperatur $T_A$ (vgl. Tabelle 3) der Wert des dynamischen Speichermoduls G' (1%, 1 Hz, 60°C) absinkt. Die Werte der Mooney Viskosität ML1+4 (100°C) nehmen zunächst ebenfalls mit zunehmender Auswurftemperatur $T_A$ entsprechend einer fortschreitenden Silanisierung der Kieselsäureoberfläche ab. R5M1 zeigt jedoch eine hohen Anstieg der Mooney Viskosität ML1+4 (100°C) gegenüber R4M1 und R3M1. R4M1 und R3M1 sind noch nicht anvernetzt, R5M1 hingegen schon.

Es zeigt sich in Tabelle 4 sowie in den Abb. 1 bis Abb. 5, dass der Schalldämpfungskoeffizient $\alpha_{rel}$ mit zunehmender Auswurfstemperatur der Kautschukmischungen entsprechend abnimmt. Die Werte des relativen Schalldämpfungskoeffizienten $\alpha_{rel}$ korrelieren dabei mit dem Speichermodul G'(1%, 1 Hz, 60°C). Der Dämpfungskoeffizient $\alpha_{rel}$ ist wie der Speichermodul G'(1%, 1 Hz, 60°C) ein Maß für die Effizienz der Silanisierung der Kieselsäureoberfläche, wenn die Kautschukmischungen R1M1 bis R5M1 bei vergleichbaren Werten für Druck und Temperatur während der Ultraschallmessung ermittelt werden. Dies ist, wie Tabelle 3 zeigt, der Fall.

Der relative Dämpfungskoeffizient $\alpha_{rel}$ des Extrudats R5M1 ist mit einem Wert von -22 [1/m] deutlich kleiner als 0 [1/m].

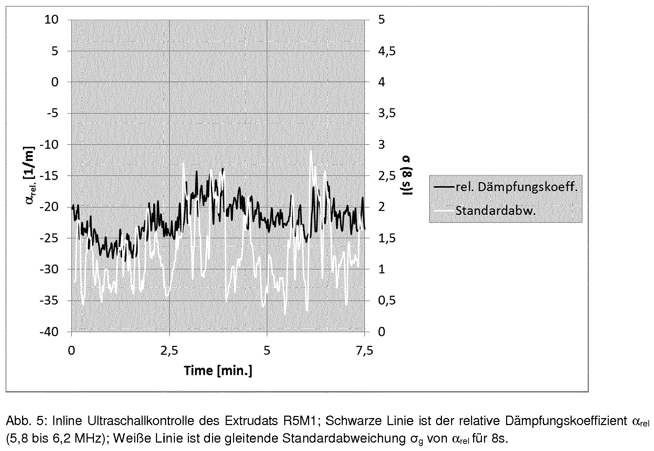

Ein weiterer sehr deutlicher Unterschied des Extrudats R5M1 gegenüber den R1M1 bis R4M1 ist die gleitende Standardabweichung $\sigma_g$ der Werte für $\alpha_{rel}$ in kleinen Zeitintervallen von < 15 s. Die Linie der Werte für $\alpha_{rel}$ des Extrudats R5M1 (Abb. 5) schwankt deutlich stärker als die Linien von R1M1 bis R4M1 (Abb. 1 bis Abb. 4). Dementsprechend ist der Mittelwert der gleitenden Standardabweichung $\sigma_g$ von $\alpha_{rel}$ für 8 s in Tabelle 4 für die Extrudate R1M1 bis R4M1 mit 0,1 m$^{-1}$ bzw. 0,2 m$^{-1}$ gering. Für R5M1 mit einer Auswurftemperatur $T_A$ von 180°C ist die gleitende Standardabweichung $\sigma_g$ von $\alpha_{rel}$ für 8 s mit 1,3 m$^{-1}$ sehr groß. R5M1 liegt anvernetzt vor, was mit der hohen Mooneyviskosität ML1+4 (100°C) von R5M1 korreliert.

Die Standardabweichung $\sigma$ des relativen Dämpfungskoeffizienten $\alpha_{rel}$ über die gesamte Zeit der Extrusion von 7,5 min beträgt für die Extrudate R1M1 bis R4M1 1 m$^{-1}$ und für R5M1 3 m$^{-1}$. Der relative Unterschied der Standardabweichungen $\sigma$ zwischen den Extrudaten R1M1 bis R4M1 und R5M1 ist bei dem langen Zeitintervall von 7,5 min allerdings deutlich geringer als der der gleitenden Standardabweichungen $\sigma_g$. Der Grund hierfür ist, dass über ein längeres Zeitintervall auch geringe Schwankungen in den Messbedingungen und/oder Extrusionsparametern zu der Standardabweichung beitragen, sodass der Wert für die Standardabweichung insgesamt zunimmt.

Die Detektion der Anvernetzung einer Kautschukmischung lässt sich demnach bevorzugt über die gleitende Standardabweichung $\sigma_g$ von $\alpha_{rel}$ über einen Zeitraum von 1 bis 100 s, besonders bevorzugt von 1 bis 50 s, ganz besonders bevorzugt von 1 bis 15 s bestimmen, hier bei 8 s.

Die vergleichbaren Werte für die Schallgeschwindigkeit $V_S$ aller Chargen R1M1 bis R5M1 belegen, dass Mischungstemperatur, Druck und Zusammensetzung der Mischungschargen einheitlich sind.

**Patentansprüche**

1. Verfahren zur Prüfung der Silanisierung von hellen Füllstoffen, wobei eine Mischung enthaltend mindestens einen silanisierten hellen Füllstoff, vorzugsweise silanisierte Kieselsäure, und mindestens einen Kautschuk mit Ultraschallwellen in einem Frequenzbereich von 4 bis 10 MHz, vorzugsweise von 5 bis 7 MHz, beschallt wird und die Signalstärke der Ultraschallwellen nach Transmission durch die Kautschukmischung bestimmt wird, wobei der relative Dämpfungskoeffizient $\alpha_{rel}$ der Kautschukmischung in dem Frequenzbereich der Ultraschallwellen bestimmt wird, die Standardabweichung $\sigma$ des relativen Dämpfungskoeffizienten $\alpha_{rel}$ ermittelt wird und $\alpha_{rel}$ und $\sigma$ zur Detektion des Anvernetzens der Kautschukmischung verwendet werden.

2. Verfahren gemäß Anspruch 1, **dadurch gekennzeichnet, dass** die Kautschukmischung bei einer Temperatur von größer als 160 °C hergestellt wird, bevorzugt bei einer Temperatur von größer als 160 °C bis 200 °C, besonders bevorzugt von größer als 160 °C bis 180 °C.

3. Verfahren gemäß einem der Ansprüche 1 oder 2, **dadurch gekennzeichnet, dass** das Anvernetzen der Kautschukmischung dadurch detektiert wird, dass die Standardabweichung $\sigma$ des relativen Dämpfungskoeffizienten $\alpha_{rel}$ der anvernetzten Kautschukmischung, bevorzugt hergestellt bei einer Temperatur von größer als 160 °C, größer als 0,5 [1/m], bevorzugt größer als 1 [1/m] ist, bevorzugt bezogen auf eine Messzeit von 1 bis 500 s, besonders bevorzugt 1 bis 100 s, ganz besonders bevorzugt 1 bis 50 s, meist bevorzugt 1 bis 15 s, und der relative Dämpfungskoeffizient $\alpha_{rel}$ der anvernetzten Kautschukmischung, bevorzugt hergestellt bei einer Temperatur von größer als 160 °C, kleiner als 0 [1/m] ist, bevorzugt kleiner als -15 [1/m].

4. Verfahren gemäß einem der Ansprüche 1 bis 3, **dadurch gekennzeichnet, dass** die gleitende Standardabweichung $\sigma_g$ des relativen Dämpfungskoeffizienten $\alpha_{rel}$ der anvernetzten Kautschukmischung, hergestellt bei einer Temperatur von größer als 160°C, größer als 1 [1/m] ist, bezogen auf eine Messzeit von 1 bis 50 s, bevorzugt von 1 bis 15 s, und der relative Dämpfungskoeffizient $\alpha_{rel}$ der anvernetzten Kautschukmischung, hergestellt bei einer Temperatur von größer als 160°C, kleiner als 0 [1/m] ist, bevorzugt kleiner als -15 [1/m].

5. Verfahren gemäß einem der Ansprüche 1 bis 4, **dadurch gekennzeichnet, dass** das Verfahren ein Verfahren zur Detektion des Anvernetzens der Kautschukmischung ist.

6. Verfahren gemäß einem der Ansprüche 1 bis 5, **dadurch gekennzeichnet, dass** der mindestens eine silanisierte helle Füllstoff aus mindestens einem hellen Füllstoff und mindestens einem Silanisierungsmittel hergestellt wird.

7. Verfahren gemäß Anspruch 6, **dadurch gekennzeichnet, dass** die Kautschukmischung weniger als 10 phr Vernetzungsmittel außer dem mindestens einen Silanisierungsmittel und dem mindestens einen silanisierten hellen Füllstoff enthält, bevorzugt weniger als 5 phr, besonders bevorzugt weniger als 2 phr.

8. Verfahren gemäß einem der Ansprüche 1 bis 3 oder 5 bis 7, **dadurch gekennzeichnet, dass** die Standardabweichung $\sigma$ die gleitende Standardabweichung $\sigma_g$ ist.

9. Verfahren gemäß einem der Ansprüche 1 bis 8, **dadurch gekennzeichnet, dass** die Mischung extrudiert und das erhaltene Extrudat beschallt wird.

10. Verfahren zur Herstellung von einer mindestens einen silanisierten hellen Füllstoff enthaltenden Kautschukmischung, wobei mindestens ein heller Füllstoff mit mindestens einem Kautschuk und mindestens einem Silanisierungsmittel gemischt wird bei einer Temperatur von größer als 160°C und wobei zumindest ein Teil der erhaltenen Kautschukmischung, vorzugsweise mehr als 1 Vol.-%, besonders bevorzugt mehr als 10 Vol.-%, ganz besonders bevorzugt mehr als 25 Vol.-% der Kautschukmischung, bezogen auf das Gesamtvolumen der Kautschukmischung, durch das Verfahren gemäß einem der Ansprüche 1 bis 9 geprüft wird.

11. Verfahren gemäß Anspruch 10, **dadurch gekennzeichnet, dass** das Verfahren zur Herstellung von einer mindestens einen silanisierten hellen Füllstoff enthaltenden Kautschukmischung bei einer Temperatur von größer als 160 °C bis 200 °C erfolgt, bevorzugt von größer als 160 °C bis 180°C.

12. Verfahren gemäß einem der Ansprüche 10 oder 11, wobei die gleitende Standardabweichung $\sigma_g$ des relativen Dämpfungskoeffizienten $\alpha_{rel}$ der Kautschukmischung kleiner als 0,5 [1/m] ist, bevorzugt kleiner als 0,3 [1/m], bezogen auf eine Messzeit von 1 bis 100 s, bevorzugt von 1 bis 50 s, besonders bevorzugt 1 bis 15 s, und der relative

Dämpfungskoeffizient $\alpha_{rel}$ der Kautschukmischung kleiner als 0 [1/m] ist, bevorzugt kleiner als -5 [1/m], besonders bevorzugt kleiner als -5 [1/m] und gleich oder größer als -15 [1/m] ist.

13. Verfahren gemäß einem der Ansprüche 1-9 oder 10-12, **dadurch gekennzeichnet, dass** der mindestens eine helle Füllstoff ausgewählt ist aus der Gruppe bestehend aus Glimmer, Kaolin, Kieselerde, Calciumcarbonate, Zinkoxid, Aluminiumoxid, Titandioxid, Kieselsäuren, Kreide und Talkum, bevorzugt Kieselsäuren und meist bevorzugt gefällte Kieselsäuren.

14. Verfahren gemäß einem der Ansprüche 6-7 oder 10-13, **dadurch gekennzeichnet, dass** das mindestens eine Silanisierungsmittel ein Silan ist, bevorzugt ausgewählt aus der Gruppe bestehend aus Bis(3-triethoxysilylpropyl)tetrasulfid, Bis(triethoxysilylpropyl)disulfid, Bis(triethoxysilylpropyl)disulfid, 3-Thiocyanatopropyltriethoxysilan, 3-Mercaptopropyl-di(tridecan-1-oxy-13-penta(ethylenoxid))ethoxysilan und Gammamercaptopropyltrimethoxysilan, besonders bevorzugt Bis(3-triethoxysilylpropyl)tetrasulfid.

15. Verfahren zur Herstellung von vernetzbaren, mindestens einen silanisierten hellen Füllstoff enthaltenden Kautschukmischungen, in dem nach der Herstellung der Kautschukmischung gemäß einem der Ansprüche 10-14 ein oder mehrere weitere Vernetzungsmittel zugefügt werden.

16. Verfahren zur Herstellung von Vulkanisaten umfassend die Herstellung von vernetzbaren, silanisierte helle Füllstoffe enthaltenden Kautschukmischungen gemäß Anspruch 15 und die nachfolgende Vulkanisation bei einer Temperatur im Bereich von 100 °C bis 200 °C.

17. Verwendung von Messvorrichtungen zur Bestimmung der Schallwellenamplitude und/oder Schallwellenintensität bei einer Frequenz der Ultraschallwellen in einem Bereich von 4 bis 10 MHz, vorzugsweise von 5 bis 7 MHz, in einem Verfahren gemäß einem der Ansprüche 1 bis 16.

Fig. 1

Fig.2

Fig.3

Abb. 1: Inline Ultraschallkontrolle des Extrudats R1M1; Schwarze Linie ist der relative Dämpfungskoeffizient $\alpha_{rel}$(5,8 bis 6,2 MHz); Weiße Linie ist die gleitende Standardabweichung $\sigma_g$ von $\alpha_{rel}$ für 8 s.

Abb. 2: Inline Ultraschallkontrolle des Extrudats R2M1; Schwarze Linie ist der relative Dämpfungskoeffizient $\alpha_{rel}$(5,8 bis 6,2 MHz); Weiße Linie ist die gleitende Standardabweichung $\sigma_g$ von $\alpha_{rel}$ für 8 s.

Abb. 3: Inline Ultraschallkontrolle des Extrudats R3M1; Schwarze Linie ist der relative Dämpfungskoeffizient $\alpha_{rel}$ (5,8 bis 6,2 MHz); Weiße Linie ist die gleitende Standardabweichung $\sigma_g$ von $\alpha_{rel}$ für 8 s.

Abb. 4: Inline Ultraschallkontrolle des Extrudats R4M1; Schwarze Linie ist der relative Dämpfungskoeffizient $\alpha_{rel}$ (5,8 bis 6,2 MHz); Weiße Linie ist die gleitende Standardabweichung $\sigma_g$ von $\alpha_{rel}$ für 8 s.

Abb. 5: Inline Ultraschallkontrolle des Extrudats R5M1; Schwarze Linie ist der relative Dämpfungskoeffizient $\alpha_{rel}$ (5,8 bis 6,2 MHz); Weiße Linie ist die gleitende Standardabweichung $\sigma_g$ von $\alpha_{rel}$ für 8s.

Europäisches
Patentamt

European
Patent Office

Office européen
des brevets

# EUROPÄISCHER RECHERCHENBERICHT

Nummer der Anmeldung

EP 19 15 7923

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 3 196 246 A1 (LANXESS DEUTSCHLAND GMBH [DE]) 26. Juli 2017 (2017-07-26) * Zusammenfassung; Ansprüche 1,2,4,8,9,11-14 * * Beispiele * * Absätze [0009], [0013], [0015], [0016], [0025] - [0033], [0042], [0050], [0051], [0082] * ----- | 1,6,7,9, 17 | INV. C08K5/00 C08K9/06 G01N29/04 C08C19/25 C08K5/5419 C08K5/54 C08K3/013 B29C48/92 |
| A | EP 0 911 359 A1 (PIRELLI [IT]) 28. April 1999 (1999-04-28) * Zusammenfassung; Anspruch 1 * * Absätze [0012], [0063], [0073], [0074] * ----- | 1-17 | ADD. C08K3/22 C08K3/26 C08K3/34 C08K3/36 |

RECHERCHIERTE
SACHGEBIETE (IPC)

C08K
C08L
G01N
C09J
C08C
B29C

Der vorliegende Recherchenbericht wurde für alle Patentansprüche erstellt

| Recherchenort | Abschlußdatum der Recherche | Prüfer |
|---|---|---|
| München | 21. August 2019 | Schütte, Maya |

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 19 15 7923

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-08-2019

| Im Recherchenbericht angeführtes Patentdokument | Datum der Veröffentlichung | Mitglied(er) der Patentfamilie | Datum der Veröffentlichung |
|---|---|---|---|
| EP 3196246 A1 | 26-07-2017 | CN 108603000 A<br>EP 3196246 A1<br>EP 3408329 A1<br>JP 2019505800 A<br>KR 20180105145 A<br>US 2019031866 A1<br>WO 2017129471 A1 | 28-09-2018<br>26-07-2017<br>05-12-2018<br>28-02-2019<br>27-09-2018<br>31-01-2019<br>03-08-2017 |
| EP 0911359 A1 | 28-04-1999 | AR 017148 A1<br>AT 263801 T<br>BR 9803366 A<br>DE 69728538 D1<br>DE 69728538 T2<br>EP 0911359 A1<br>ES 2219745 T3<br>JP 4346707 B2<br>JP H11179785 A | 22-08-2001<br>15-04-2004<br>14-12-1999<br>13-05-2004<br>17-03-2005<br>28-04-1999<br>01-12-2004<br>21-10-2009<br>06-07-1999 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 69728538 T2
- EP 0911359 A
- EP 1357156 A
- WO 2017129471 A1
- EP 2314442 A
- EP 2009058041 W
- DE 102008040138 A


**In der Beschreibung aufgeführte Nicht-Patentliteratur**

- **F. RÖTHEMEYER ; F. SOMMER.** Kautschuktechnologie Werkstoffe, Verarbeitung Produkte. Hanser Verlag, 272
- **F. RÖTHEMEYER ; F. SOMMER.** Kautschuktechnologie Werkstoffe, Verarbeitung Produkte. Hanser Verlag
- **F. RÖTHEMEYER ; F. SOMMER.** Kautschuktechnologie Werkstoffe, Verarbeitung Produkte. Hanser Verlag, 390
- **HOCHREIN et al.** *Plastverarbeiter,* September 2009, 92
- **A. SCHRÖDER ; L. GRÄFF ; L. WAWRZINSKI.** *Rubber World,* 2015, vol. 251, 28
- *Rubber World,* 2015, vol. 251, 28
- **M. JAUNICH ; B. STARK ; B. HOSTER.** *Polymer Testing,* 2009, vol. 28, 84